# EUROPEAN PATENT SPECIFICATION

(11) **EP 2 365 986 B1**
(45) Date of publication and mention of the grant of the patent: **15.07.2015**
(21) Application number: 09759889.0
(22) Date of filing: 18.11.2009
(51) Int. Cl.: C07K 16/18, C07K 16/34

(54) **DEGLYCOSYLATED ANTIBODIES**
DEGLYKOSYLIERTE ANTIKÖRPER
ANTICORPS DÉGLYCOSYLÉS

(30) Priority: 18.11.2008 GB 0821100
(43) Date of publication of application: 21.09.2011
(73) Proprietor: Hansa Medical AB, 220 07 Lund (SE)
(72) Inventor: COLLIN, Mattias, 224 71 Lund (SE); ALLHORN, Maria, 256 57 Ramlosa (SE); HOLMDAHL, Rikard, 113 61 Stockholm (SE); NANDAKUMAR, Kutty, Selva, 754 26 Uppsala (SE)
(74) Representative: Roques, Sarah Elizabeth
(86) International application number: PCT/EP2009/008215
(87) International publication number: WO 2010/057626

(56) References cited:
- WO-A-2008/071418
- COLLIN M ET AL: "IgG glycan hydrolysis by a bacterial enzyme as a therapy against autoimmune conditions" PROCEEDINGS OF THE NATIONAL ACADEMY OF SCIENCES OF USA, NATIONAL ACADEMY OF SCIENCE, WASHINGTON, DC, US, vol. 105, no. 11, 18 March 2008 (2008-03-18), pages 4265-4270, XP002486740 ISSN: 0027-8424
- ALBERT HEIKE ET AL: "In vivo enzymatic modulation of IgG glycosylation inhibits autoimmune disease in an IgG subclass-dependent manner." PROCEEDINGS OF THE NATIONAL ACADEMY OF SCIENCES OF THE UNITED STATES OF AMERICA 30 SEP 2008, vol. 105, no. 39, 30 September 2008 (2008-09-30), pages 15005-15009, XP002569095 ISSN: 1091-6490
- SCANLAN CHRISTOPHER N ET AL: "Making autoantibodies safe." PROCEEDINGS OF THE NATIONAL ACADEMY OF SCIENCES OF THE UNITED STATES OF AMERICA 18 MAR 2008, vol. 105, no. 11, 18 March 2008 (2008-03-18), pages 4081-4082, XP002569096 ISSN: 1091-6490
- NANDAKUMAR ET AL: "Therapeutic cleavage of IgG: new avenues for treating inflammation" TRENDS IN IMMUNOLOGY, ELSEVIER, RAHWAY, NJ, US, vol. 29, no. 4, 6 March 2008 (2008-03-06), pages 173-178, XP022575981 ISSN: 1471-4906
- HERMAN S ET AL: "Molecular mechanisms of inflammatory bone damage: emerging targets for therapy" TRENDS IN MOLECULAR MEDICINE, ELSEVIER CURRENT TRENDS, vol. 14, no. 6, 1 June 2008 (2008-06-01), pages 245-253, XP022711882 ISSN: 1471-4914 [retrieved on 2008-05-09]
- NAPARSTEK Y ET AL: "The role of autoantibodies in autoimmune disease.", ANNUAL REVIEW OF IMMUNOLOGY 1993, vol. 11, 1993, pages 79-104, XP004171825, ISSN: 0732-0582
- ALLHORN MARIA ET AL: "Human IgG/Fc gamma R interactions are modulated by streptococcal IgG glycan hydrolysis", PLOS ONE, PUBLIC LIBRARY OF SCIENCE, SAN FRANCISCO, CA; US, vol. 3, no. 1, 1 January 2008 (2008-01-01) , pages 1-12, XP002486739, ISSN: 1932-6203, DOI: 10.1371/JOURNAL.PONE.0001413
- NANDAKUMAR KUTTY SELVA ET AL: "Dominant suppression of inflammation by glycan-hydrolyzed IgG.", PROCEEDINGS OF THE NATIONAL ACADEMY OF SCIENCES OF THE UNITED STATES OF AMERICA 18 JUN 2013, vol. 110, no. 25, 18 June 2013 (2013-06-18), pages 10252-10257, ISSN: 1091-6490
- S. JOLLES ET AL: 'Clinical uses of intravenous immunoglobulin' CLINICAL & EXPERIMENTAL IMMUNOLOGY vol. 142, no. 1, 01 October 2005, pages 1 - 11, XP055064000 DOI: 10.1111/j.1365-2249.2005.02834.x ISSN: 0009-9104
- SAPIR T ET AL: "Facing the enigma of immunomodulatory effects of intravenous immunoglobulin", CLINICAL REVIEWS IN ALLERGY AND IMMUNOLOGY, HUMANA PRESS, TOTOWA, NJ, US, vol. 29, no. 3, 1 December 2005 (2005-12-01), pages 185-200, XP002568002, ISSN: 1080-0549, DOI: 10.1385/CRIAI:29:3:185

### SEQUENCE LISTING

<110> HANSA MEDICAL AB
<120> DEGLYCOSYLATED ANTIBODIES
<130> N105092A SER/GML
<150> GB 0821100.5
<151> 2008-11-18
<160> 3
<170> PatentIn version 3.0
<210> 1
<211> 959
<212> PRT
<213> Streptococcus pyogenes
<400> 1
   Glu Glu Lys Thr Val Gln Val Gln Lys Gly Leu Pro Ser Ile Asp Ser
   1               5                   10                  15
   Leu His Tyr Leu Ser Glu Asn Ser Lys Lys Glu Phe Lys Glu Glu Leu
               20                  25                  30
   Ser Lys Ala Gly Gln Glu Ser Gln Lys Val Lys Glu Ile Leu Ala Lys
           35                  40                  45
   Ala Gln Gln Ala Asp Lys Gln Ala Gln Glu Leu Ala Lys Met Lys Ile
       50                  55                  60
   Pro Glu Lys Ile Pro Met Lys Pro Leu His Gly Pro Leu Tyr Gly Gly
   65                  70                  75                  80
   Tyr Phe Arg Thr Trp His Asp Lys Thr Ser Asp Pro Thr Glu Lys Asp
                   85                  90                  95
   Lys Val Asn Ser Met Gly Glu Leu Pro Lys Glu Val Asp Leu Ala Phe
               100                 105                 110
   Ile Phe His Asp Trp Thr Lys Asp Tyr Ser Leu Phe Trp Lys Glu Leu
           115                 120                 125
   Ala Thr Lys His Val Pro Lys Leu Asn Lys Gln Gly Thr Arg Val Ile
       130                 135                 140
   Arg Thr Ile Pro Trp Arg Phe Leu Ala Gly Gly Asp Asn Ser Gly Ile
   145                 150                 155                 160
   Ala Glu Asp Thr Ser Lys Tyr Pro Asn Thr Pro Glu Gly Asn Lys Ala
                   165                 170                 175
   Leu Ala Lys Ala Ile Val Asp Glu Tyr Val Tyr Lys Tyr Asn Leu Asp
               180                 185                 190
   Gly Leu Asp Val Asp Val Glu His Asp Ser Ile Pro Lys Val Asp Lys
           195                 200                 205
   Lys Glu Asp Thr Ala Gly Val Glu Arg Ser Ile Gln Val Phe Glu Glu
       210                 215                 220
   Ile Gly Lys Leu Ile Gly Pro Lys Gly Val Asp Lys Ser Arg Leu Phe
   225                 230                 235                 240
   Ile Met Asp Ser Thr Tyr Met Ala Asp Lys Asn Pro Leu Ile Glu Arg
                   245                 250                 255
   Gly Ala Pro Tyr Ile Asn Leu Leu Leu Val Gln Val Tyr Gly Ser Gln
               260                 265                 270
   Gly Glu Lys Gly Gly Trp Glu Pro Val Ser Asn Arg Pro Glu Lys Thr
           275                 280                 285
   Met Glu Glu Arg Trp Gln Gly Tyr Ser Lys Tyr Ile Arg Pro Glu Gln
       290                 295                 300
   Tyr Met Ile Gly Phe Ser Phe Tyr Glu Glu Asn Ala Gln Glu Gly Asn
   305                 310                 315                 320
   Leu Trp Tyr Asp Ile Asn Ser Arg Lys Asp Glu Asp Lys Ala Asn Gly
                   325                 330                 335
   Ile Asn Thr Asp Ile Thr Gly Thr Arg Ala Glu Arg Tyr Ala Arg Trp
               340                 345                 350
   Gln Pro Lys Thr Gly Gly Val Lys Gly Gly Ile Phe Ser Tyr Ala Ile
           355                 360                 365
   Asp Arg Asp Gly Val Ala His Gln Pro Lys Lys Tyr Ala Lys Gln Lys
       370                 375                 380
   Glu Phe Lys Asp Ala Thr Asp Asn Ile Phe His Ser Asp Tyr Ser Val
   385                 390                 395                 400
   Ser Lys Ala Leu Lys Thr Val Met Leu Lys Asp Lys Ser Tyr Asp Leu
                   405                 410                 415
   Ile Asp Glu Lys Asp Phe Pro Asp Lys Ala Leu Arg Glu Ala Val Met
               420                 425                 430
   Ala Gln Val Gly Thr Arg Lys Gly Asp Leu Glu Arg Phe Asn Gly Thr
           435                 440                 445
   Leu Arg Leu Asp Asn Pro Ala Ile Gln Ser Leu Glu Gly Leu Asn Lys
       450                 455                 460
   Phe Lys Lys Leu Ala Gln Leu Asp Leu Ile Gly Leu Ser Arg Ile Thr
   465                 470                 475                 480
   Lys Leu Asp Arg Ser Val Leu Pro Ala Asn Met Lys Pro Gly Lys Asp
                   485                 490                 495
   Thr Leu Glu Thr Val Leu Glu Thr Tyr Lys Lys Asp Asn Lys Glu Glu
               500                 505                 510
   Pro Ala Thr Ile Pro Pro Val Ser Leu Lys Val Ser Gly Leu Thr Gly
           515                 520                 525
   Leu Lys Glu Leu Asp Leu Ser Gly Phe Asp Arg Glu Thr Leu Ala Gly
       530                 535                 540
   Leu Asp Ala Ala Thr Leu Thr Ser Leu Glu Lys Val Asp Ile Ser Gly
   545                 550                 555                 560
   Asn Lys Leu Asp Leu Ala Pro Gly Thr Glu Asn Arg Gln Ile Phe Asp
                   565                 570                 575
   Thr Met Leu Ser Thr Ile Ser Asn His Val Gly Ser Asn Glu Gln Thr
               580                 585                 590
   Val Lys Phe Asp Lys Gln Lys Pro Thr Gly His Tyr Pro Asp Thr Tyr
           595                 600                 605
   Gly Lys Thr Ser Leu Arg Leu Pro Val Ala Asn Glu Lys Val Asp Leu
       610                 615                 620
   Gln Ser Gln Leu Leu Phe Gly Thr Val Thr Asn Gln Gly Thr Leu Ile
   625                 630                 635                 640
   Asn Ser Glu Ala Asp Tyr Lys Ala Tyr Gln Asn His Lys Ile Ala Gly
                   645                 650                 655
   Arg Ser Phe Val Asp Ser Asn Tyr His Tyr Asn Asn Phe Lys Val Ser
               660                 665                 670
   Tyr Glu Asn Tyr Thr Val Lys Val Thr Asp Ser Thr Leu Gly Thr Thr
           675                 680                 685
   Thr Asp Lys Thr Leu Ala Thr Asp Lys Glu Glu Thr Tyr Lys Val Asp
       690                 695                 700
   Phe Phe Ser Pro Ala Asp Lys Thr Lys Ala Val His Thr Ala Lys Val
   705                 710                 715                 720
   Ile Val Gly Asp Glu Lys Thr Met Met Val Asn Leu Ala Glu Gly Ala
                   725                 730                 735
   Thr Val Ile Gly Gly Ser Ala Asp Pro Val Asn Ala Arg Lys Val Phe
               740                 745                 750
   Asp Gly Gln Leu Gly Ser Glu Thr Asp Asn Ile Ser Leu Gly Trp Asp
           755                 760                 765
   Ser Lys Gln Ser Ile Ile Phe Lys Leu Lys Glu Asp Gly Leu Ile Lys
       770                                     775 780
   His Trp Arg Phe Phe Asn Asp Ser Ala Arg Asn Pro Glu Thr Thr Asn
   785                 790                 795                 800
   Lys Pro Ile Gln Glu Ala Ser Leu Gln Ile Phe Asn Ile Lys Asp Tyr
                   805                 810                 815
   Asn Leu Asp Asn Leu Leu Glu Asn Pro Asn Lys Phe Asp Asp Glu Lys
               820                 825                 830
   Tyr Trp Ile Thr Val Asp Thr Tyr Ser Ala Gln Gly Glu Arg Ala Thr
           835                 840                 845
   Ala Phe Ser Asn Thr Leu Asn Asn Ile Thr Ser Lys Tyr Trp Arg Val
       850                 855                 860
   Val Phe Asp Thr Lys Gly Asp Arg Tyr Ser Ser Pro Val Val Pro Glu
   865                 870                 875                 880
   Leu Gln Ile Leu Gly Tyr Pro Leu Pro Asn Ala Asp Thr Ile Met Lys
                   885                 890                 895
   Thr Val Thr Thr Ala Lys Glu Leu Ser Gln Gln Lys Asp Lys Phe Ser
               900                 905                 910
   Gln Lys Met Leu Asp Glu Leu Lys Ile Lys Glu Met Ala Leu Glu Thr
           915                 920                 925
   Ser Leu Asn Ser Lys Ile Phe Asp Val Thr Ala Ile Asn Ala Asn Ala
       930                 935                 940
   Gly Val Leu Lys Asp Cys Ile Glu Lys Arg Gln Leu Leu Lys Lys
   945                 950                 955
<210> 2
<211> 995
<212> PRT
<213> Streptococcus pyogenes
<400> 2
   Met Asp Lys His Leu Leu Val Lys Arg Thr Leu Gly Cys Val Cys Ala
   1               5                   10                  15
   Ala Thr Leu Met Gly Ala Ala Leu Ala Thr His His Asp Ser Leu Asn
               20                  25                  30
   Thr Val Lys Ala Glu Glu Lys Thr Val Gln Val Gln Lys Gly Leu Pro
           35                  40                  45
   Ser Ile Asp Ser Leu His Tyr Leu Ser Glu Asn Ser Lys Lys Glu Phe
       50                  55                  60
   Lys Glu Glu Leu Ser Lys Ala Gly Gln Glu Ser Gln Lys Val Lys Glu
   65                  70                  75                  80
   Ile Leu Ala Lys Ala Gln Gln Ala Asp Lys Gln Ala Gln Glu Leu Ala
                   85                  90                  95
   Lys Met Lys Ile Pro Glu Lys Ile Pro Met Lys Pro Leu His Gly Pro
               100                 105                 110
   Leu Tyr Gly Gly Tyr Phe Arg Thr Trp His Asp Lys Thr Ser Asp Pro
           115                 120                 125
   Thr Glu Lys Asp Lys Val Asn Ser Met Gly Glu Leu Pro Lys Glu Val
       130                 135                 140
   Asp Leu Ala Phe Ile Phe His Asp Trp Thr Lys Asp Tyr Ser Leu Phe
   145                 150                 155                 160
   Trp Lys Glu Leu Ala Thr Lys His Val Pro Lys Leu Asn Lys Gln Gly
                   165                 170                 175
   Thr Arg Val Ile Arg Thr Ile Pro Trp Arg Phe Leu Ala Gly Gly Asp
               180                 185                 190
   Asn Ser Gly Ile Ala Glu Asp Thr Ser Lys Tyr Pro Asn Thr Pro Glu
           195                 200                 205
   Gly Asn Lys Ala Leu Ala Lys Ala Ile Val Asp Glu Tyr Val Tyr Lys
   210 215 220
   Tyr Asn Leu Asp Gly Leu Asp Val Asp Val Glu His Asp Ser Ile Pro
   225 230 235 240
   Lys Val Asp Lys Lys Glu Asp Thr Ala Gly Val Glu Arg Ser Ile Gln
   245 250 255
   Val Phe Glu Glu Ile Gly Lys Leu Ile Gly Pro Lys Gly Val Asp Lys
   260 265 270
   Ser Arg Leu Phe Ile Met Asp Ser Thr Tyr Met Ala Asp Lys Asn Pro
   275 280 285
   Leu Ile Glu Arg Gly Ala Pro Tyr Ile Asn Leu Leu Leu Val Gln Val
   290 295 300
   Tyr Gly Ser Gln Gly Glu Lys Gly Gly Trp Glu Pro Val Ser Asn Arg
   305 310 315 320
   Pro Glu Lys Thr Met Glu Glu Arg Trp Gln Gly Tyr Ser Lys Tyr Ile
   325 330 335
   Arg Pro Glu Gln Tyr Met Ile Gly Phe Ser Phe Tyr Glu Glu Asn Ala
   340 345 350
   Gln Glu Gly Asn Leu Trp Tyr Asp Ile Asn Ser Arg Lys Asp Glu Asp
   355 360 365
   Lys Ala Asn Gly Ile Asn Thr Asp Ile Thr Gly Thr Arg Ala Glu Arg
   370 375 380
   Tyr Ala Arg Trp Gln Pro Lys Thr Gly Gly Val Lys Gly Gly Ile Phe
   385 390 395 400
   Ser Tyr Ala Ile Asp Arg Asp Gly Val Ala His Gln Pro Lys Lys Tyr
   405 410 415
   Ala Lys Gln Lys Glu Phe Lys Asp Ala Thr Asp Asn Ile Phe His Ser
   420 425 430
   Asp Tyr Ser Val Ser Lys Ala Leu Lys Thr Val Met Leu Lys Asp Lys
   435 440 445
   Ser Tyr Asp Leu Ile Asp Glu Lys Asp Phe Pro Asp Lys Ala Leu Arg
   450 455 460
   Glu Ala Val Met Ala Gln Val Gly Thr Arg Lys Gly Asp Leu Glu Arg
   465 470 475 480
   Phe Asn Gly Thr Leu Arg Leu Asp Asn Pro Ala Ile Gln Ser Leu Glu
   485 490 495
   Gly Leu Asn Lys Phe Lys Lys Leu Ala Gln Leu Asp Leu Ile Gly Leu
   500 505 510
   Ser Arg Ile Thr Lys Leu Asp Arg Ser Val Leu Pro Ala Asn Met Lys
   515 520 525
   Pro Gly Lys Asp Thr Leu Glu Thr Val Leu Glu Thr Tyr Lys Lys Asp
   530 535 540
   Asn Lys Glu Glu Pro Ala Thr Ile Pro Pro Val Ser Leu Lys Val Ser
   545 550 555 560
   Gly Leu Thr Gly Leu Lys Glu Leu Asp Leu Ser Gly Phe Asp Arg Glu
   565 570 575
   Thr Leu Ala Gly Leu Asp Ala Ala Thr Leu Thr Ser Leu Glu Lys Val
   580 585 590
   Asp Ile Ser Gly Asn Lys Leu Asp Leu Ala Pro Gly Thr Glu Asn Arg
   595 600 605
   Gln Ile Phe Asp Thr Met Leu Ser Thr Ile Ser Asn His Val Gly Ser
   610 615 620
   Asn Glu Gln Thr Val Lys Phe Asp Lys Gln Lys Pro Thr Gly His Tyr
   625 630 635 640
   Pro Asp Thr Tyr Gly Lys Thr Ser Leu Arg Leu Pro Val Ala Asn Glu
   645 650 655
   Lys Val Asp Leu Gln Ser Gln Leu Leu Phe Gly Thr Val Thr Asn Gln
   660 665 670
   Gly Thr Leu Ile Asn Ser Glu Ala Asp Tyr Lys Ala Tyr Gln Asn His
   675 680 685
   Lys Ile Ala Gly Arg Ser Phe Val Asp Ser Asn Tyr His Tyr Asn Asn
   690 695 700
   Phe Lys Val Ser Tyr Glu Asn Tyr Thr Val Lys Val Thr Asp Ser Thr
   705 710 715 720
   Leu Gly Thr Thr Thr Asp Lys Thr Leu Ala Thr Asp Lys Glu Glu Thr
   725 730 735
   Tyr Lys Val Asp Phe Phe Ser Pro Ala Asp Lys Thr Lys Ala Val His
   740 745 750
   Thr Ala Lys Val Ile Val Gly Asp Glu Lys Thr Met Met Val Asn Leu
   755 760 765
   Ala Glu Gly Ala Thr Val Ile Gly Gly Ser Ala Asp Pro Val Asn Ala
   770 775 780
   Arg Lys Val Phe Asp Gly Gln Leu Gly Ser Glu Thr Asp Asn Ile Ser
   785 790 795 800
   Leu Gly Trp Asp Ser Lys Gln Ser Ile Ile Phe Lys Leu Lys Glu Asp
   805 810 815
   Gly Leu Ile Lys His Trp Arg Phe Phe Asn Asp Ser Ala Arg Asn Pro
   820 825 830
   Glu Thr Thr Asn Lys Pro Ile Gln Glu Ala Ser Leu Gln Ile Phe Asn
   835 840 845
   Ile Lys Asp Tyr Asn Leu Asp Asn Leu Leu Glu Asn Pro Asn Lys Phe
   850 855 860
   Asp Asp Glu Lys Tyr Trp Ile Thr Val Asp Thr Tyr Ser Ala Gln Gly
   865 870 875 880
   Glu Arg Ala Thr Ala Phe Ser Asn Thr Leu Asn Asn Ile Thr Ser Lys
   885 890 895
   Tyr Trp Arg Val Val Phe Asp Thr Lys Gly Asp Arg Tyr Ser Ser Pro
   900 905 910
   Val Val Pro Glu Leu Gln Ile Leu Gly Tyr Pro Leu Pro Asn Ala Asp
   915 920 925
   Thr Ile Met Lys Thr Val Thr Thr Ala Lys Glu Leu Ser Gln Gln Lys
   930 935 940
   Asp Lys Phe Ser Gln Lys Met Leu Asp Glu Leu Lys Ile Lys Glu Met
   945 950 955 960
   Ala Leu Glu Thr Ser Leu Asn Ser Lys Ile Phe Asp Val Thr Ala Ile
   965 970 975
   Asn Ala Asn Ala Gly Val Leu Lys Asp Cys Ile Glu Lys Arg Gln Leu
   980 985 990
   Leu Lys Lys
   995
<210> 3
<211> 3403
<212> DNA
<213> Streptococcus pyogenes
<400> 3
   ctcttttgtc ctgccatgga tggcaggttg gcaaaaaaat gagaaaagcc taaaaacctt  60
   aaatttgtgt tattttacct atactttgta ccttgttttt tttaataaag tgttgttata  120
   cttaaggcga actataggaa tgcgcttaca tggatggtat atcaactggg aagccatgac  180
   ttagtaccaa aaataaggag tgtccaaatg gataaacatt tgttggtaaa aagaacacta  240
   gggtgtgttt gtgctgcaac gttgatggga gctgccttag cgacccacca tgattcactc  300
   aatactgtaa aagcggagga gaagactgtt caggttcaga aaggattacc ttctatcgat  360
   agcttgcatt atctgtcaga gaatagcaaa aaagaattta aagaagaact ctcaaaagcg  420
   gggcaagaat ctcaaaaggt caaagagata ttagcaaaag ctcagcaggc agataaacaa  480
   gctcaagaac ttgccaaaat gaaaattcct gagaaaatac cgatgaaacc gttacatggt  540
   cctctctacg gtggttactt tagaacttgg catgacaaaa catcagatcc aacagaaaaa  600
   gacaaagtta actcgatggg agagcttcct aaagaagtag atctagcctt tattttccac  660
   gattggacaa aagattatag ccttttttgg aaagaattgg ccaccaaaca tgtgccaaag  720
   ttaaacaagc aagggacacg tgtcattcgt accattccat ggcgtttcct agctgggggt  780
   gataacagtg gtattgcaga agataccagt aaatacccaa atacaccaga gggaaataaa  840
   gctttagcca aagctattgt tgatgaatat gtttataaat acaaccttga tggcttagat  900
   gtggatgttg aacatgatag tattccaaaa gttgacaaaa aagaagatac agcaggcgta  960
   gaacgctcta ttcaagtgtt tgaagaaatt gggaaattaa ttggaccaaa aggtgttgat 1020
   aaatcgcggt tatttattat ggatagcacc tacatggctg ataaaaaccc attgattgag 1080
   cgaggagctc cttatattaa tttattactg gtacaggtct atggttcaca aggagagaaa 1140
   ggtggttggg agcctgtttc taatcgacct gaaaaaacaa tggaagaacg atggcaaggt 1200
   tatagcaagt atattcgtcc tgaacaatac atgattggtt tttctttcta tgaggaaaat 1260
   gctcaagaag ggaatctttg gtatgatatt aattctcgca aggacgagga caaagcaaat 1320
   ggaattaaca ctgacataac tggaacgcgt gccgaacggt atgcaaggtg gcaacctaag 1380
   acaggtgggg ttaagggagg tatcttctcc tacgctattg accgagatgg tgtagctcat 1440
   caacctaaaa aatatgctaa acagaaagag tttaaggacg caactgataa catcttccac 1500
   tcagattata gtgtctccaa ggcattaaag acagttatgc taaaagataa gtcgtatgat 1560
   ctgattgatg agaaagattt cccagataag gctttgcgag aagctgtgat ggcgcaggtt 1620
   ggaaccagaa aaggtgattt ggaacgtttc aatggcacat tacgattgga taatccagcg 1680
   attcaaagtt tagaaggtct aaataaattt aaaaaattag ctcaattaga cttgattggc 1740
   ttatctcgca ttacaaagct cgaccgttct gttttacccg ctaatatgaa gccaggcaaa 1800
   gataccttgg aaacagttct tgaaacctat aaaaaggata acaaagaaga acctgctact 1860
   atcccaccag tatctttgaa ggtttctggt ttaactggtc tgaaagaatt agatttgtca 1920
   ggttttgacc gtgaaacctt ggctggtctt gatgccgcta ctctaacgtc tttagaaaaa 1980
   gttgatattt ctggcaacaa acttgatttg gctccaggaa cagaaaatcg acaaattttt 2040
   gatactatgc tatcaactat cagcaatcat gttggaagca atgaacaaac agtgaaattt 2100
   gacaagcaaa aaccaactgg gcattaccca gatacctatg ggaaaactag tctgcgctta 2160
   ccagtggcaa atgaaaaagt tgatttgcaa agccagcttt tgtttgggac tgtgacaaat 2220
   caaggaaccc taatcaatag cgaagcagac tataaggctt accaaaatca taaaattgct 2280
   ggacgtagct ttgttgattc aaactatcat tacaataact ttaaagtttc ttatgagaac 2340
   tataccgtta aagtaactga ttccacattg ggaaccacta ctgacaaaac gctagcaact 2400
   gataaagaag agacctataa ggttgacttc tttagcccag cagataagac aaaagctgtt 2460
   catactgcta aagtgattgt tggtgacgaa aaaaccatga tggttaattt ggcagaaggc 2520
   gcaacagtta ttggaggaag tgctgatcct gtaaatgcaa gaaaggtatt tgatgggcaa 2580
   ctgggcagtg agactgataa tatctcttta ggatgggatt ctaagcaaag tattatattt 2640
   aaattgaaag aagatggatt aataaagcat tggcgtttct tcaatgattc agcccgaaat 2700
   cctgagacaa ccaataaacc tattcaggaa gcaagtctac aaatttttaa tatcaaagat 2760
   tataatctag ataatttgtt ggaaaatccc aataaatttg atgatgaaaa atattggatt 2820
   actgtagata cttacagtgc acaaggagag agagctactg cattcagtaa tacattaaat 2880
   aatattacta gtaaatattg gcgagttgtc tttgatacta aaggagatag atatagttcg 2940
   ccagtagtcc ctgaactcca aattttaggt tatccgttac ctaacgccga cactatcatg 3000
   aaaacagtaa ctactgctaa agagttatct caacaaaaag ataagttttc tcaaaagatg 3060
   cttgatgagt taaaaataaa agagatggct ttagaaactt ctttgaacag taagattttt 3120
   gatgtaactg ctattaatgc taatgctgga gttttgaaag attgtattga gaaaaggcag 3180
   ctgctaaaaa aataaacaaa gtaactttct tagatagcaa cattcagatt aaattaacaa 3240
   aatgtgacta tgataaaggt ttgctggaat tgattaacca aaagactaaa aatctgagat 3300
   gaatagtccc agatttttag tcttttatag gttttgatga cataaagcta aataatcgtt 3360
   agactaccag aaagggcgct tgtccgtgag acatggctgt ctt                   3403

## Description

### Field of the Invention

The present invention relates to intravenous immunoglobulin (IVIG) preparations which have modified glycosylation, and methods of preparing same.

### Background of the Invention

A large number of diseases and conditions involve unwanted inflammatory conditions. One illustration of a condition involving a damaging inflammatory condition is Rheumatoid Arthritis (RA). RA is a crippling and widespread disease, involving joint inflammation and damage. A variety of antibodies against self-antigens are found in RA sufferers, including antibodies against Type II Collagen. It is believed that such antibodies play an important role in the condition, with administration of monoclonal antibodies against Type II collagen being able to induce arthritis in a mouse model of the disease. There is a need for ways to treat conditions such as RA by reducing or eliminating inflammatory condition underlying the disorders.

Joller et al (2005) Clin Exp Immunol 142, p.11 describes the clinical users of intravenous immunoglobulin (IVIG) preparations.

Collin et al (2008) PNAS 105, p.4265 and Albert et al (2008) PNAS 105, p.15005 disclose the use of EndoS to reduce the pathogenic effects of antoantibodies.

### Summary of the Invention

The present invention provides an intravenous immunoglobulin (IVIG) preparation which has been treated with an EndoS enzyme wherein the glycosylation of the EndoS-treated IVIG preparation consists only of the first N-acetylglucosamine residue with an optional fucose group.

The present invention also provides an *in vitro* method of producing an intravenous immunoglobulin (IVIG) preparation comprising treating said IVIG preparation with an EndoS enzyme wherein the glycosylation of the EndoS-treated IVIG preparation consists only of the first N-acetylglucosamine residue with an optional fucose group.

### Brief description of Figures

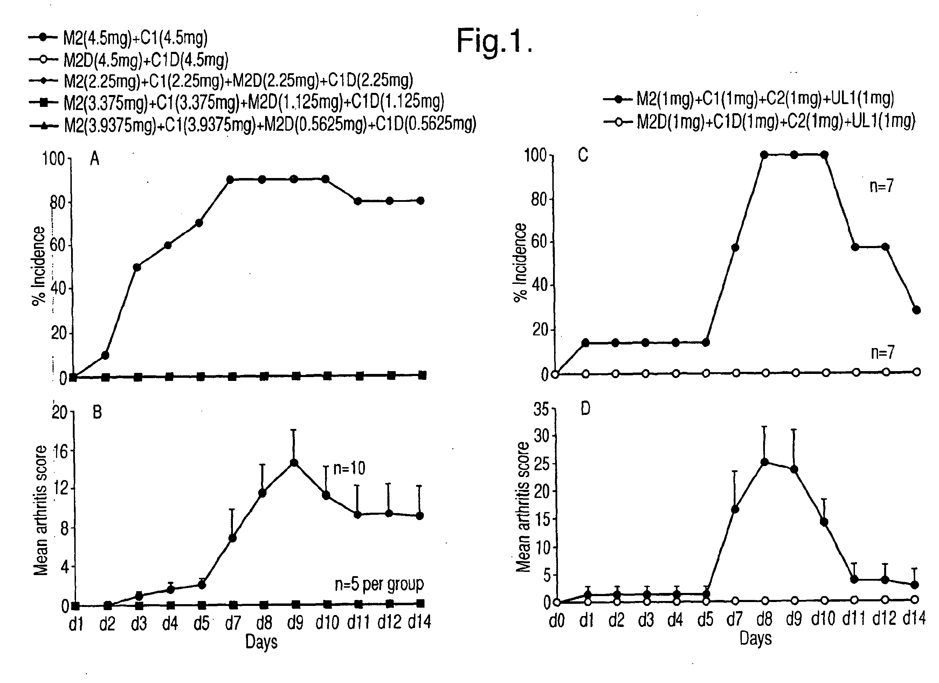

Figure 1, panels (A) and (B) shows how anti-CII antibodies with modified glycosylation dominantly suppress induction of CAIA (Collagen Antigen Induced Arthritis). Mice were administered: (i) fully glycosylated monoclonal antibodies; (ii) the same monoclonal antibodies, but with modified glycosylation; or (iii) mixtures of both at differing ratios of glycosylated to modified forms. Panel (A) shows arthritis incidence and panel (B) mean arthritis score of arthritic animals only, with n indicating the number of mice used in each group.
Figure 1, panels (C) and (D) shows that antibodies with modified glycosylation are capable of dominantly suppressing induction of arthritis by monoclonal antibodies recognising different epitopes to the antibodies with modified glycosylation. The closed circles show the results for a mixture of four different monoclonal antibodies in glycosylated form, with the open circles showing the results for a mixture where two of the four antibodies have modified glycosylation. Panel (C) shows arthritis incidence and panel (D) mean arthritis score of arthritic animals only, with n- indicating the number of mice used in each group.

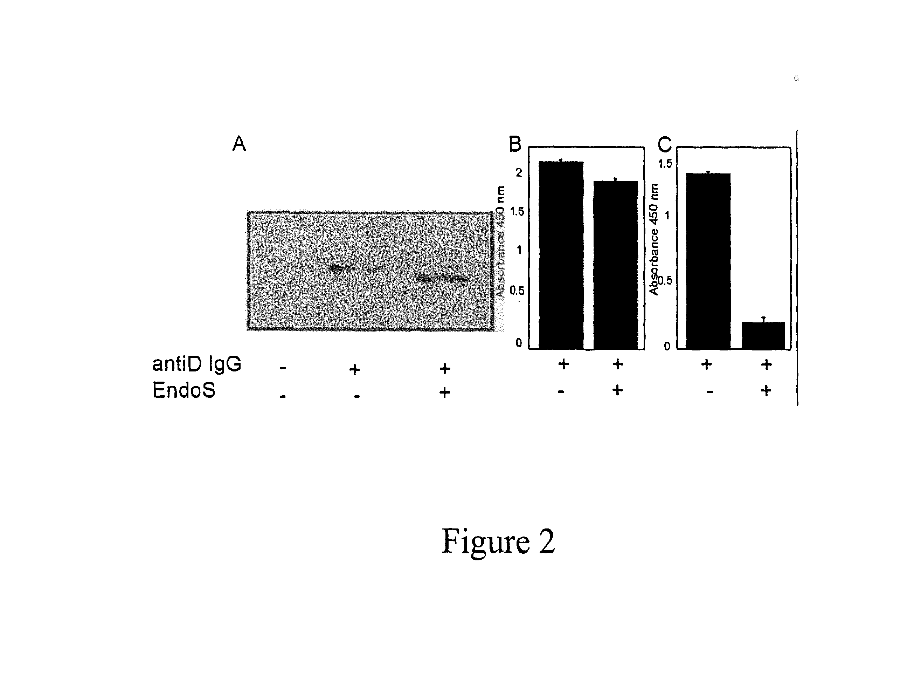

Figure 2 shows hydrolysis of human anti-D IgG by EndoS. Panel (A) shows quantification of RBC-bound IgG by solubilisation of sensitized RBC followed by Western blot using antiserum against human IgG. Panel (B) shows quantification of the amount of RBC-bound IgG by ELISA using antiserum against human IgG. Panel (C) shows glycan hydrolysis of RBC-bound IgG determined by lectin ELISA. For each panel the presence or absence of anti-D and EndoS in the test sample is indicated at the bottom.

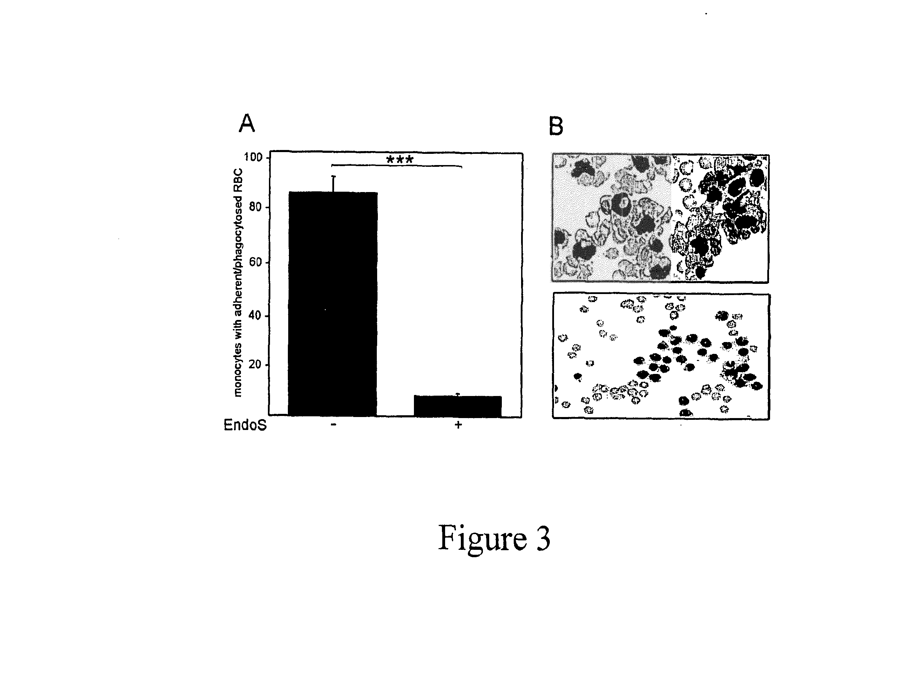

Figure 3 shows that red blood cells sensitised with anti-D IgG pre-treated with EndoS are not phagocytosed by monocytes. Panel (A) shows the degree of adherence and phagocytosis determined by monocyte monolayer assay (MMA) and expressed as the number monocytes with one or more adherent/phagocytosed RBC out of 100 monocytes detected. Panel (B) shows the appearance of "rosettes" showed using Grünvald-Giemsa staining of monocytes monolayers incubated with RBC sensitized with anti-D at the top and below the results obtained when the anti-D was pre-treated with EndoS.

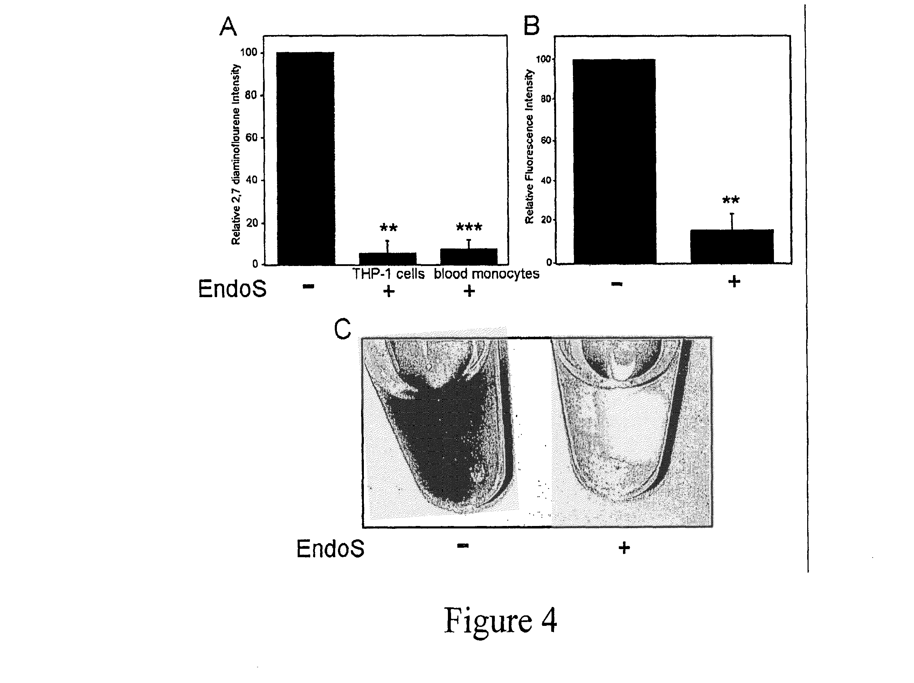

Figure 4 shows intracellular ingestion of anti-D sensitized RBC and accumulation of haemoglobin is inhibited by pre-treatment of anti-D with EndoS. Panel (A) shows ingestion of sensitized RBC by THP-1 cells and blood monocytes presented as the amount of internalized haemoglobin detected by reactivity with 2,7-diaminofluorene. Panel (B) shows the degree of ingested RBC determined by measuring the fluorescence as measured via purified blood monocytes being incubated with FITC-labelled sensitized RBC. Panel (C) shows resuspended monocytes after incubation with anti-D sensitized RBC followed by the removal of uningested RBC, with the left photo showing RBC sensitized with anti-D and the right, anti-D pre-treated with EndoS.

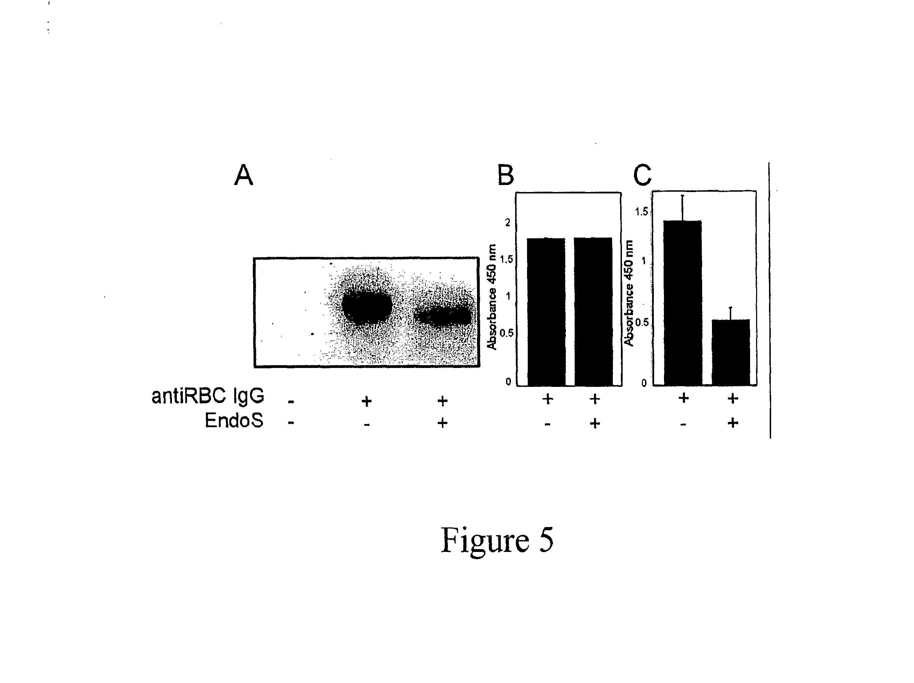

Figure 5 shows hydrolysis of anti-human RBC-IgG by EndoS. Panel (A) shows Western blot results for RBC bound IgG, where rabbit antisera against human RBC treated with EndoS or PBS was used for sensitization of RBC and bound IgG detected using antiserum against rabbit IgG. Panel (B) shows the amount of RBC-bound antibody as quantified by ELISA using antiserum against rabbit IgG. Panel (C) shows glycan hydrolysis of RBC-bound IgG as determined by lectin ELISA.

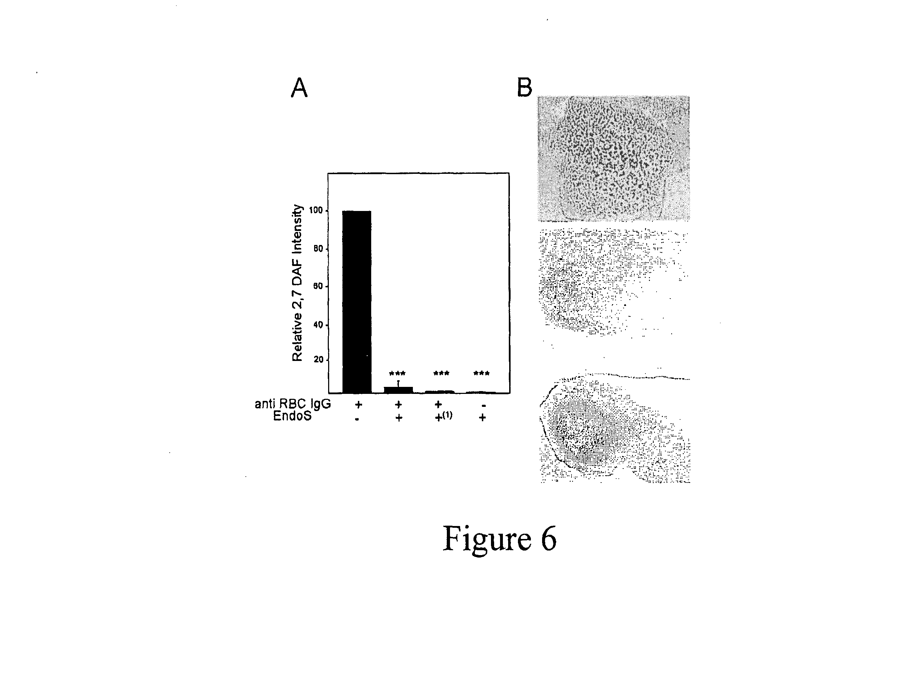

Figure 6 shows EndoS inhibits hemolysis and agglutination of RBC sensitized with anti-RBC IgG. Panel (A) shows haemoglobin content quantified using 2,7-diaminofluorene staining. Rabbit IgG was directed against human RBC, pre-treated with EndoS or not and added to whole blood and after one hour incubation the blood cells were pelleted and supernatants analyzed for haemoglobin. For (1), instead of the pre-treatment of antibody, EndoS was directly added to blood and incubated one hour. The relative amount of haemoglobin is presented as a mean of four experiments and standard error of the mean. Panel (B) shows C1q deposition into sensitized RBC detected using flourescein-conjugated anti-human C1q. Anti-RBC IgG, 80 µg, was added to 700 µl human blood, diluted 35 times in PBS and incubated 1 hour at 37° C.

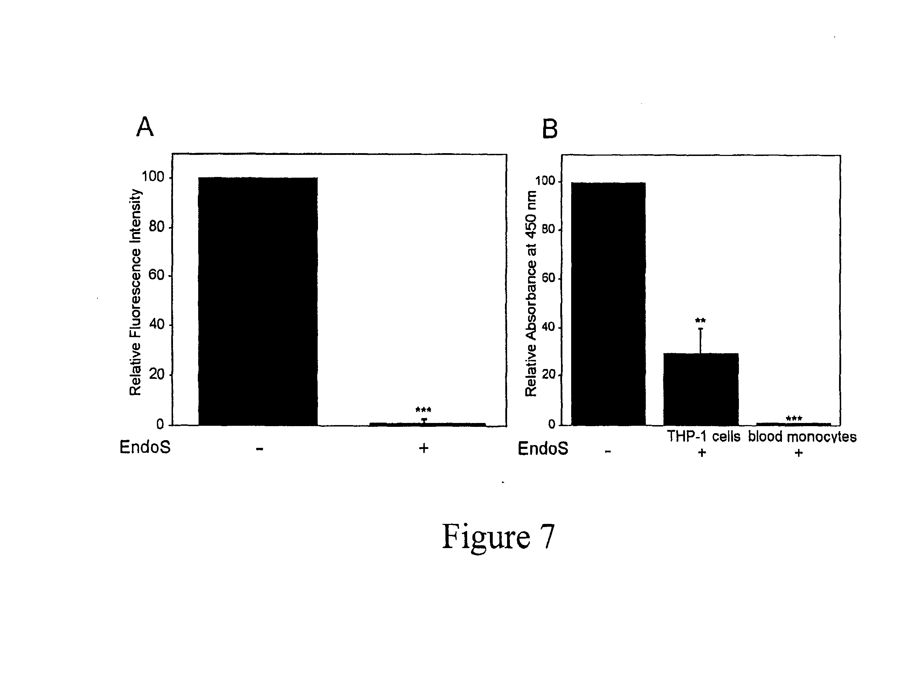

Figure 7, panel (A) shows EndoS inhibiting binding of C1q to IgG-sensitized RBC and secretion of IL-8 by monocytes induced by sensitized RBC. Human serum was added to RBC freshly sensitized with rabbit antisera against human RBC, with or without pretreatment of antibody with EndoS. C1q deposition into sensitized RBC was detected using flourescein-conjugated anti-human C1q. Panel (B) shows IL-8 content. THP-1 cells or monocytes were purified from human blood were incubated with sensitized RBC, with or without pre-treatment of anti-D with EndoS, for 3 hours and the supernatants analyzed for IL-8.

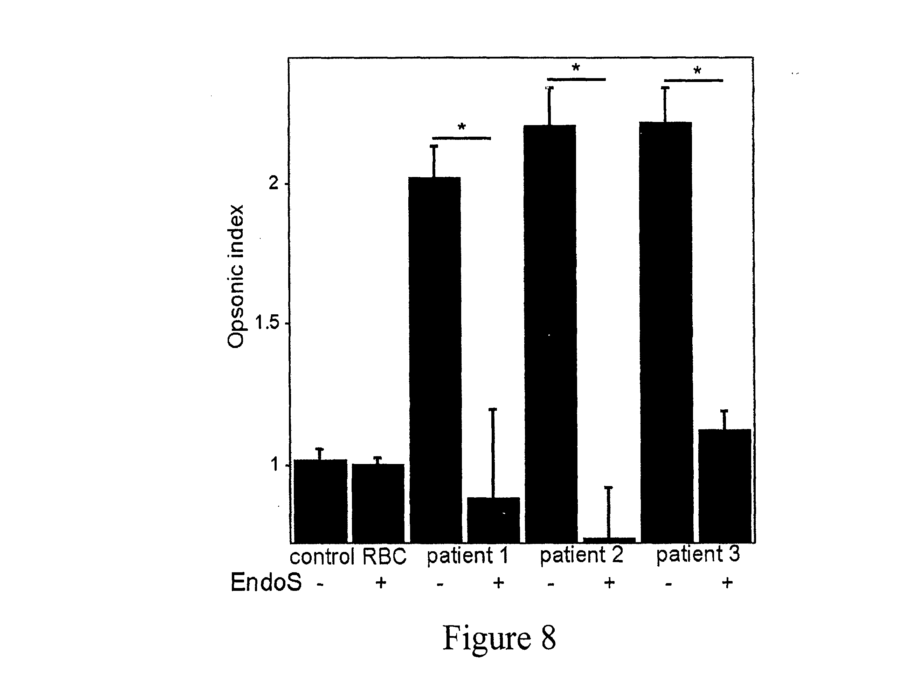

Figure 8 shows treatment of RBC sensitized with human immune plasma with EndoS inhibits oxygen metabolite production by monocytes exposed to sensitized RBC. RBC were sensitized with DAT positive human plasma from three patients and subsequently treated with EndoS before addition to purified blood monocytes. The secretion of oxygen metabolites from monocytes was detected by measuring the chemiluminescence.

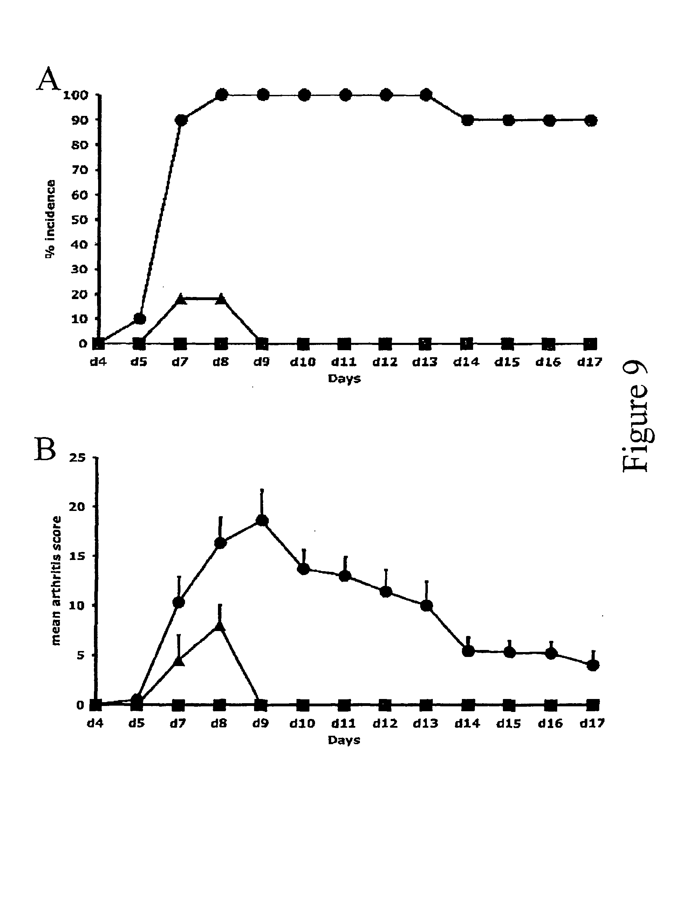

Figure 9 panels (A) and (B) shows that antibodies with modified glycosylation are capable of dominantly suppressing induction of arthritis by monoclonal antibodies recognising overlapping or non-overlapping epitopes to the antibodies with modified glycosylation. The triangles show the results for mice treated with a pair of antibodies having modified glycosylation which recognise epitopes that do not overlap with the epitopes recognised by the arthritogenic antibodies. The squares show the results for mice treated with a pair of antibodies having modified glycosylation which recognise epitopes that do overlap with the epitopes recognised by the arthritogenic antibodies. The circles show the results for mice which were not treated with antibodies having modified glycosylation. Panel (A) shows arthritis incidence and panel (B) mean arthritis score of arthritic animals only. n=11 for the non-overlapping epitope group and the no treatment group. n=5 for the overlapping epitope group.

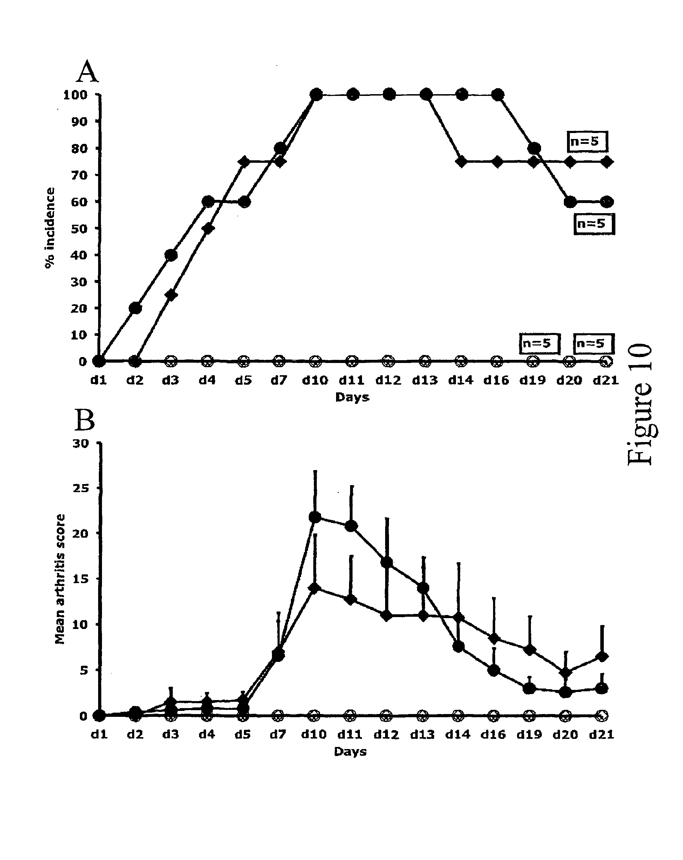

Figure 10 panels (A) and (B) shows that antibodies with modified glycosylation are capable of dominantly suppressing induction of arthritis in animals with and without a spleen. The open diamonds show animals which underwent splenectomy and were treated with a pair of antibodies having modified glycosylation. The open circles show animals which underwent sham splenectomy and were treated with a pair of antibodies having modified glycosylation. The closed diamonds show animals which underwent splenectomy and were untreated. The closed circles show animals which underwent sham splenectomy and were untreated. Panel (A) shows arthritis incidence and panel (B) mean arthritis score of arthritic animals only. n indicates the number of animals in each group.

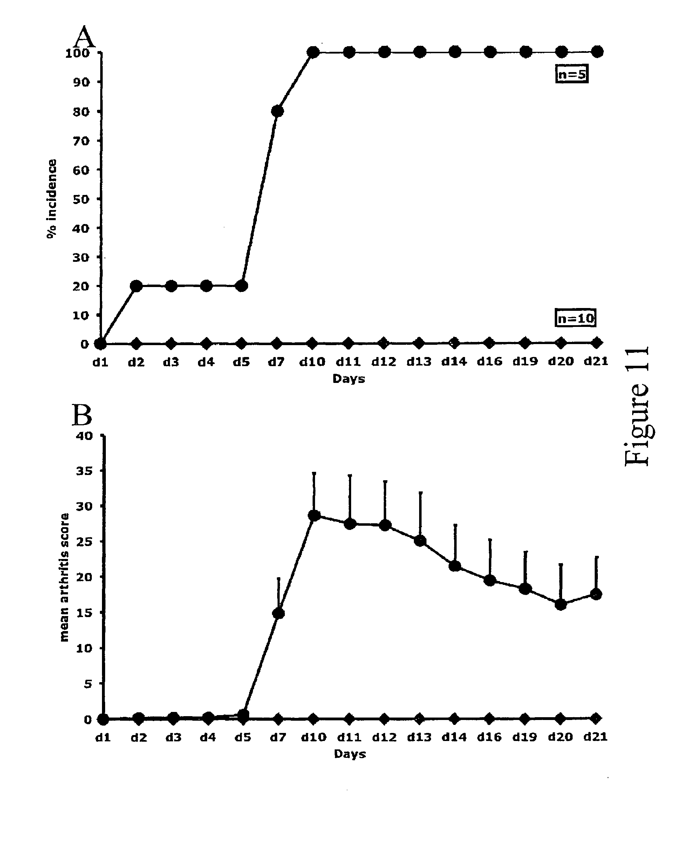

Figure 11 panels (A) and (B) shows that antibodies with modified glycosylation are capable of dominantly suppressing arthritis independent of antigen specificity. The closed diamonds show animals which were treated with a pair of antibodies having modified glycosylation, said antibodies being specific for different antigens to the disease-inducing antibodies. The closed circles show animals which were untreated. Panel (A) shows arthritis incidence and panel (B) mean arthritis score of arthritic animals only. It indicates the number of animals in each group.

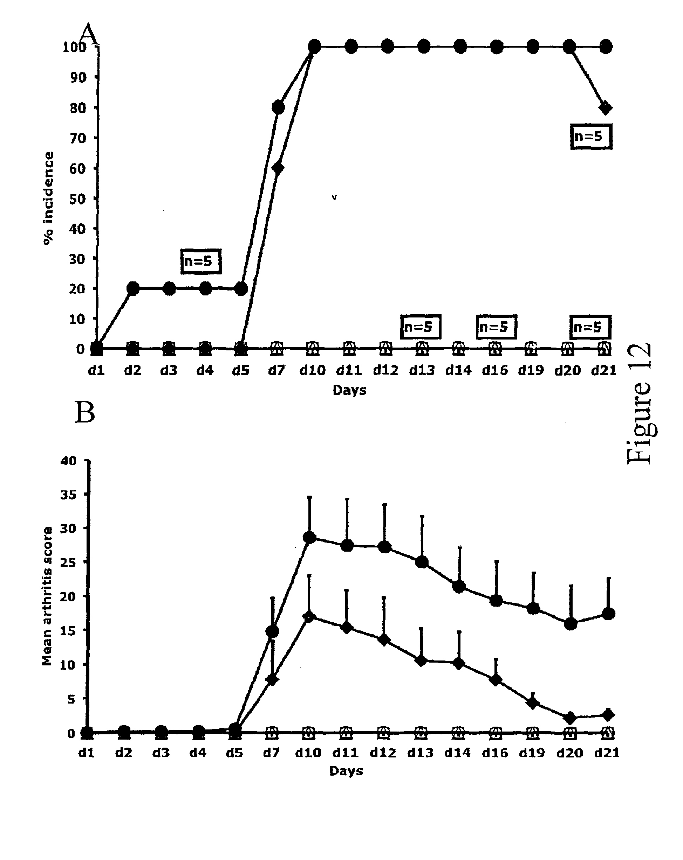

Figure 12 panels (A) and (B) shows that a single antibody with modified glycosylation is capable of dominantly suppressing arthritis at even low dose. The closed diamonds show animals which were treated with a single antibody having modified glycosylation (M2139D) at 50µg, the open squares show animals which were treated with M2139D at 250µg, the open circles show animals which were treated with M2139D at 1000µg, and the open triangles show animals which were treated with M2139D at 4000µg. The closed circles show animals which were untreated. Panel (A) shows arthritis incidence and panel (B) mean arthritis score of arthritic animals only. n indicates the number of animals in each group.

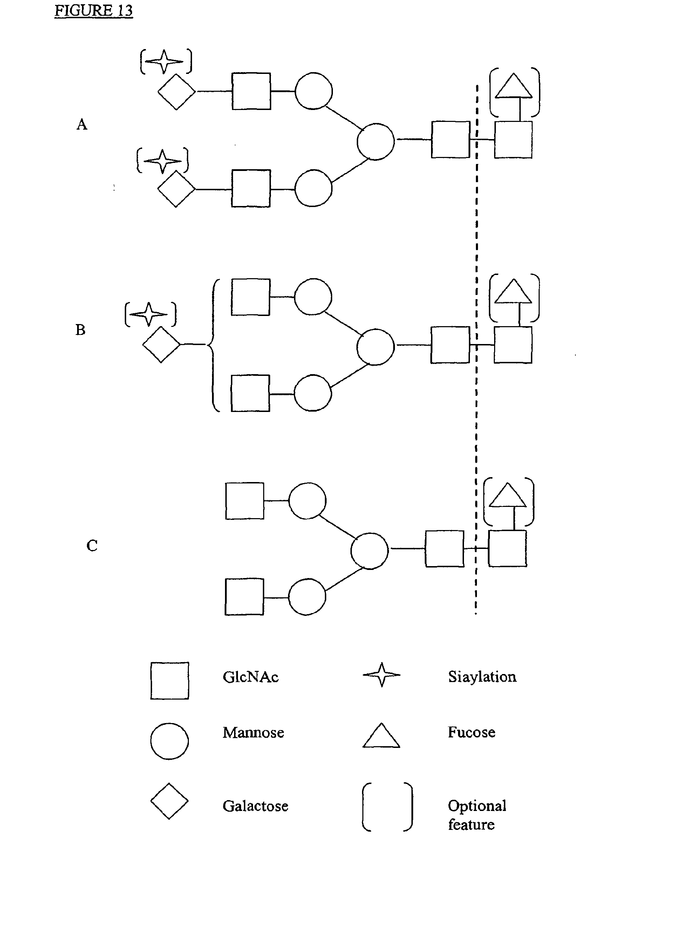

Figure 13 shows a schematic representation of the glycosyl chains present at Asn-297 of the heavy chains of naturally occurring IgG molecules. This population of glycan chains contains three sets of glycoforms termed IgG-G0, -G1 and -G2. The IgG-G2 (Figure 13A) biantennary glycans at Asn-297 have two arms that terminate in galactose residues. Approximately 16% of total IgG glycosyl chains take this form. The IgG-G1 (Figure 13B) glycans lack a terminal galactose residue on one biantennary arm. Approximately 35% of total IgG glycosyl chains take this form. The IgG-G0 (Figure 13C) glycans lack a terminal galactose residue on both biantennary arms. Approximately 35% of total IgG glycosyl chains take this form. Another 14% of total IgG glycosyl chains consists of IgG-G1 or IgG-G2 forms which are sialylated. Further variety is introduced, in that some glycans also have a bisecting GlcNAc residue connected to the first mannose residue (approximately 30% of the total IgG1 glycan pool), a core fucose attached to the first core GlcNAc residue (approximately 70% of the total IgG1 glycan pool), and sialylation of the terminal 1,3 arm galactose residues (approximately 14% of the total IgG1 glycan pool). The dashed line indicates the position at which EndoS cleaves these structures.

### Brief Description of the Sequences

SEQ ID NO: 1 is an amino acid sequence of EndoS from *S. pyogenes* AP1.
SEQ ID NO: 2 is an amino acid sequence of EndoS from *S. pyogenes* AP1, including a signal sequence.
SEQ ID NO: 3 is a nucleic acid sequence encoding EndoS from *S. pyogenes* AP1, including a signal sequence.

### Detailed Description of the Invention

The present invention relates to intravenous immunoglobulin (IVIG) preparations which have modified glycosylation, and methods of preparing same wherein the glycosylation of the EndoS-treated IVIG preparation consists only of the first N-acetylglucosamine residue with an optional fucose group.

Also disclosed herein is the use of antibodies in general, or antibody fragments, with modified glycosylation. The antibodies and fragments display the ability to suppress an inflammatory condition forming part of the disease or condition to be treated or prevented. In some instances, the disease or condition may comprise, consist of, or consist essentially of, the inflammatory condition.

Furthermore, where corresponding antibodies, or antibody fragments, with normal glycosylation have been previously employed, those of the invention with modified glycosylation display increased potency and/or reduced side-effects.

Disclosed herein is an antibody, or antibody fragment, with modified glycosylation for use in treating or preventing a disease or condition, where the antibody or fragment: (a) suppresses an inflammatory condition forming part of the disease or condition to be treated or prevented; and/or (b) displays increased efficacy and/or decreased side effects in comparision to treatment or prevention with the corresponding antibody with unmodified glycosylation. The antibody or antibody fragment may therefore be used to suppress inflammatory conditions. A number of inflammatory conditions involve antibodies in a pivotal role.

### Antibodies

The term "antibody", as referred to herein, includes whole antibodies and any antigen binding fragment *(i.e.,* "antigen-binding portion") or single chains thereof. In one preferred instance, the antibody or fragment is monoclonal. In another it is polyclonal.

An antibody refers to a protein comprising at least two heavy (H) chains and two light (L) chains inter-connected by disulfide bonds, or an antigen binding portion thereof. Each heavy chain is comprised of a heavy chain variable region (abbreviated herein as VH) and a heavy chain constant region. Each light chain is comprised of a light chain variable region (abbreviated herein as VL) and a light chain constant region.

The variable regions of the heavy and light chains contain a binding domain that interacts with an antigen. The VH and VL regions can be further subdivided into regions of hypervariability, termed complementarity determining regions (CDR), interspersed with regions that are more conserved, termed framework regions (FR). The constant regions of the antibodies may mediate the binding of the immunoglobulin to host tissues or factors, including various cells of the immune system (e.g., effector cells) and the first component (Clq) of the classical complement system.

The antibody, or fragment, disclosed herein has modified glycosylation. That is the antibody or fragment does not have the normal glycosylation of an antibody of that class. The modification of glycosylation may be the result of:
(i) modification of the natural glycosylation; and/or
(ii) the antibody being synthesised without normal glycosylation.

Reference herein to modified glycosylation encompasses an absence of glycosylation as well as the presence of a different glycosyl group to normal or the presence of an alternative chemical entity. In one preferred instance, the modification is the retention of a partial glycosyl group, particularly a GlcNAc residue with or without a linked fucose group. The presence of fucose linked to the core GlcNAc residue of complex biantennary glysoyl groups is a physiological varation. A substantial fraction (approx 30%) of circulating antibodies lack this fucose.

Thus, in one instance, the antibody or fragment lacks full glycosylation. For instance, whilst it may retain a partial glycosyl group, it does not have the full glycosyl group found on the normal antibody. Such a glycosyl group may be referred to as a truncated glycosyl group. The modification may be enzymatic cleavage, in particular leaving a partial or truncated glycosyl group, particularly a GlcNAc residue with or without linked fucosegroup, which may be referred to as a GlcNac(-Fucose) stump.

Modification of glycosylation may have been achieved by any suitable means, for instance chemically or enzymatically and in particular enzymatically. In a particularly preferred instance, the antibody or fragment will have been treated using the enzyme EndoS, including any of the forms of EndoS described herein. Treatment with Endo S leaves only the first *N*-acetylglucosamine (GlcNAc) residue with a linked fucose group (if a fucose group was originally present), from the usual glycosyl group, as cleavage has occurred between the two core GlcNAc residues. Reference to modification herein encompasses in particular such a modification and in one particularly preferred instance, the antibody or fragment of the invention comprises such a residual group. Any means of achieving such modifications, or the presence of such a group, may be employed whether chemical or enzymatic. In one instance, the modified group is one obtainable by, preferably obtained by, EndoS cleavage.

In a further instance, the sequence of the antibody or fragment may have been altered so that the natural glycosylation of the antibody does not occur or is modified. Alternatively, the antibody or fragment may have been synthesised in a system that does not result in full glycosylation. Thus, it may be that glycosylation has been prevented, for instance through the use of an inhibitor of the enzyme or enzymes responsible for glycosylation or via synthesis in a system where the enzymes are lacking naturally or have been eliminated using such techniques as knocking out encoding genes. In some systems only partial glycosylation may occur because not all of the enzymes necessary for full glycosylation are present.

In normally glycosylated antibodies, the oligosaccharide is typically of the complex biantennary type comprising two core *N*-acetylglucosamine (GlcNAc) residues, in which a core fucose may or may not be linked to the innermost *N-*acetylglucosamine (GlcNAc). These glycans are typically located in the interface between the C_{H}2 domains (second constant domain of the heavy chains). In IgG antibodies glycosylation typically occurs in each heavy chain at Asn-297.

That is, for IgG molecules there is typically a glycosyl group attached to the residue at position 297 of each heavy chain. Examples of the naturally occurring glycosyl groups present at Asn-297 in IgG molecules are shown in Figure 13. Equivalent or similar glycosylation sites exist in other antibody classes. For example, N-linked glycosylation sites exist in IgM molecules at Asn-171, Asn-332, Asn-395, Asn-402 and Asn-563. All except Asn-402 and Asn-563 are commonly occupied by complex biantennary glycans. N-linked glycosylation sites also exist in IgA molecules at Asn-263 in the CH2 domain, and Asn-459, in IgD molecules at Asn-354, Asn-445 and Asn-496, and in IgE molecules at Asn-140, Asn-168, Asn-218, Asn-365, Asn-371, Asn-383 and Asn-394. The glycosyl groups normally present at these sites are preferably complex biantennary glycans.

The glycosylation in such positions is typically an N-linked glycosyl group, but may be O-linked if a suitable amino acid is present in the relevant position.

In a preferred instance, the antibody or fragment lacks the normal full glycosylation at a position in one and preferably both of the heavy chains, which position in a normally glycosylated antibody is linked to a complex biantennary glycosyl group comprising two core GlcNAc residues. That is, only a partial or truncated glycosyl chain is linked to said position. In a particularly preferred instance, the antibody is an IgG antibody, and the position is 297 (normally Asn-297) or a corresponding position.

In a particularly preferred instance, the normal glycosyl group is absent and only the first *N*-acetylglucosamine (GlcNAc) residue remains, with or without the linked fucose group. In other words, the truncated glycosyl group may consist of a single core GlcNAc residue, optionally linked to a fucose residue. Thus, the normal glycosyl chain may have been cleaved by hydrolysing the linkage between the two GlcNAc residues. That represents the cleavage resulting from treatment with EndoS and in a preferred instance the antibody or fragment of the invention has been treated with EndoS to modify the glycosylation of the antibody or fragment. In alternative embodiments, the Asn-297 residue has been substituted or deleted so that glycosylation no longer occurs.

In one instance, the normal glycosyl group is absent and only the first two GlcNAc residues remain, the first GlcNAc residue preferably with its normally linked fucose group (if present). In other words, the truncated glycosyl group may consist of a first core GlcNAc residue linked to a second core GlcNAc residue, wherein the first core GlcNAc residue is optionally linked to a fucose residue. Thus, the normal glycosyl chain may have been cleaved by hydrolysing the linkage between the second GlcNAc residue and mannose.

In one instrance, the normal glycosyl group is absent and only the first two GlcNAc residues and first mannose residue remain. The first GlcNAc residue preferably has its normally linked fucose group (if present). The mannose residue may optionally have be linked to a single further GlcNAc residue, typically by an α1,4 linkage. In other words, the truncated glycosyl group may consist of a first core GlcNAc residue linked to a second core GlcNAc residue linked to a mannose residue, wherein the first core GlcNAc residue is optionally linked to a fucose residue, and/or wherein the mannose residue is optionally linked to a further, single GlcNAc residue. Thus, the normal glycosyl chain may have been cleaved by hydrolysing the linkages between the first mannose residue and the branching mannose residues attached to it. These linkages are typically α1,3 and α1,6 linkages.

In the above instances, one or more GlcNAc residues may be replaced with a N-Acetylgalactosamine (GalNAc) residue.

Alternatively, modification may mean that the glycosyl group is replaced with a different chemical entity. In one instance, the glycosyl group may have been replaced with a monosaccharide or disaccharide, an aliphatic group or compound, an aromatic group or compound, an amino acid or amino acid derivative, a nucleotide or nucleotide derivative, a lipid or lipid derivative, any organic group, or any combination thereof, which results in an antibody or fragment which (a) suppresses an inflammatory condition forming part of the disease or condition to be treated or prevented; and/or (b) displays increased efficacy and/or decreased side effects in comparision to treatment or prevention with the corresponding antibody with unmodified glycosylation. Such an antibody or fragment may typically be an agonist to an antibody or fragment which has modified glycosylation such that the normal glycosylation has been replaced with a truncated glycosyl group consisting of a single core GlcNAc residue, optionally linked to a fucose residue.

In one instance, the modification of glycosylation may mean that the antibody may be unable to activate complement. In a further instance, the antibody or fragment may modify Fc receptor binding. In some embodiments, the antibody or fragment may modify both functions. In some instances, the antibody or fragment may retain the ability to bind to inhibitory FcR but lack binding to activatory FcR. In others, it may not. In other instances the modification of glycosylation may affect the binding for other unknown receptors.

The antibody may be of any antibody class. For instance, it may be IgA, IgD, IgE, IgM or IgG. In an especially preferred embodiment the antibody, or fragment, is IgG or a fragment thereof. The antibody may be any subtype, for instance it may be any of IgG₁, IgG₂, IgG₃ or IgG₄. The antibody, may in some instances be monomeric, dimeric or pentameric, in a particularly preferred instance it may be monomeric.

Preferably, the antibody is one in which an amino acid position in one or both heavy chains is normally linked to a complex biantennary glycosyl group comprising two core GlcNAc residues. Preferably, the amino acid normally occurring at this position is retained in one or both heavy chains, but the glcosyl group attached to one or both is modified. In a preferred instance, the antibody or fragment lacks the normal full glycosylation at the said position in one and preferably both of the heavy chains. That is, only a partial or truncated glycosyl chain is linked to the position.

The amino acid normally present in the position is typically Asn, but may be any alternative amino acid to which a glycosyl group can be linked, or may be substituted for such an alternative amino acid. Suitable amino acids include Ser, Thr or Asp. Where Ser or Thr are present, the linked glycosyl group may typically be O-linked rather than N-linked. In such instances, one or more GlcNAc residues, typically at least the first core GlcNAc residue, may be replaced with a GalNAc residue.

As explained above, the residue at position 297 (normally Asn-297) in IgG molecules is a preferred example of a position which in a normally glycosylated antibody is linked to a complex biantennary glycosyl group comprising two core GlcNAc residues. Thus, for example, the Asn residue at position 297 in IgG molecules may be replaced with a different amino acid such as Ser, Thr or Asp. Hence the modification in the case of IgG may be N297S or N297T or N297D.

Alternatively, the glycosyl group may be linked to a different position relative to a normally glycosylated antibody, provided that it is possible to link a glycosyl group to the different position. That is, the modified glycosylation of the present invention may be linked to a position corresponding to, but not identical to, a glycosylated position in a normally glycosylated antibody. For example, in IgG molecules, the modified glycosylation may be linked to any one of positions 287 to 296, or 298 to 308, provided that a suitable amino acid is present at said position. Suitable amino acids include in particular Asn, Asp, Ser and Thr. Thus, as a specific example, an Asn residue at position 289 (Asn-289) may be considered to be in a position corresponding to Asn-297.

As used herein, the term "epitope" generally refers to the site on a target antigen that is recognised by an antibody. It may be a short peptide derived from or as part of a protein. However the term is also intended to include peptides with glycopeptides and carbohydrate epitopes. A single antigenic molecule may comprise several different epitopes. Epitopes can be identified from knowledge of the amino acid and corresponding DNA sequences of the peptide, as well as from the nature of particular amino acids (e.g., size, charge, etc.) and the codon dictionary, without undue experimentation. See, e.g., Ivan Roitt, Essential Immunology, 1988; Janis Kuby, Immunology, 1992 e.g., pp. 79-81. In one instance, the binding epitope of a disease causing antibody may be determined in the subject to be treated.

The location of an epitope may be identified by routine methods. For example, the general location of an epitope may be determined by assessing the ability of an antibody to bind to different fragments or variant polypeptides derived from the target. The specific amino acids within the target that make contact with an antibody may also be determined using routine methods. For example, the antibody and target molecule may be combined and the antibody/target complex may be crystallised. The crystal structure of the complex may be determined and used to identify specific sites of interaction between the antibody and its target.

An antibody is preferably capable of binding to its target with an affinity that is at least two-fold, 10-fold, 50-fold, 100-fold or greater than its affinity for binding to another non-target molecule.

An antibody may, for instance, be a monoclonal antibody or a polyclonal antibody. In one preferred embodiment, an antibody is a monoclonal antibody. An antibody may be a chimeric antibody, a CDR-grafted antibody, a human or humanised antibody or an antigen-binding portion of any thereof. For the production of both monoclonal and polyclonal antibodies, the experimental animal is suitably a mammal such as a goat, rabbit, rat or mouse.

Polyclonal antibodies are antibodies that are derived from different B cell lines. A polyclonal antibody may comprise a mixture of different immunoglobulin molecules that are directed against a specific antigen. The polyclonal antibody may comprise a mixture of different immunoglobulin molecules that bind to one or more different epitopes within an antigen molecule. Polyclonal antibodies may be produced by routine methods such as immunisation of a suitable animal, with the antigen of interest. Blood may be subsequently removed from the animal and the immunoglobulin fraction purified. Modification of glycosylation may then be performed.

Monoclonal antibodies are immunoglobulin molecules that are identical to each other and have a single binding specificity and affinity for a particular epitope. Monoclonal antibodies (mAbs) of the present invention can be produced by a variety of techniques, including conventional monoclonal antibody methodology e.g., the standard somatic cell hybridization technique of Kohler and Milstein (1975) Nature 256: 495, or viral or oncogenic transformation of B lymphocytes. The preferred animal system for preparing hybridomas is the murine system. Hybridoma production in the mouse is a very well-established procedure. Immunization protocols and techniques for isolation of immunized splenocytes for fusion are known in the art. Fusion partners (e.g., murine myeloma cells) and fusion procedures are also known.

To generate hybridomas producing monoclonal antibodies, splenocytes and/or lymph node cells from immunized mice can be isolated and fused to an appropriate immortalized cell line, such as a mouse myeloma cell line. The resulting hybridomas can be screened for the production of antigen-specific antibodies. The antibody secreting hybridomas can be replated, screened again, and if still positive for suitable IgG, the monoclonal antibodies can be subcloned at least twice by limiting dilution. The stable subclones can then be cultured *in vitro* to generate small amounts of antibody in tissue culture medium for characterization.

Antibody fragments may be employed. Any suitable antibody fragment may be employed, typically that, when administered, displays reduced side effects, whilst retaining the ability to act as a therapeutic and/or the ability to reduce or ameliorate an immune response in a disease or condition, particularly to suppress an inflammatory condition. Anywhere reference is made herein to the use of an antibody, a functional antibody fragment may also be employed unless the context dictates specifically otherwise.

Preferably, the antibody fragment will retain the residue which is normally linked to a complex biantennary glycosyl group comprising two core GlcNAc residues (for example the Asn-297 residue in IgG), but will have modified glycosylation. Alternatively, it may retain the region comprising said residue, but the specific residue has been substituted or deleted as explained above. The fragment may or may not be capable of binding to antigen. The antibody or fragment may lack an antigen-binding portion. For example, the fragment may be an Fc fragment.

The term "antigen-binding portion" of an antibody refers to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include a Fab fragment, a F(ab')₂ fragment, a Fab' fragment, a Fd fragment, a Fv fragment, a dAb fragment and an isolated complementarity determining region (CDR). Single chain antibodies such as scFv and heavy chain antibodies such as VHH and camel antibodies are also intended to be encompassed within the term "antigen-binding portion" of an antibody. These antibody fragments may be obtained using conventional techniques known to those of skill in the art, and the fragments may be screened for utility in the same manner as intact antibodies.

In one preferred instance, the fragments will lack the ability to bind C1q and/or Fc receptors, but still possess the regions responsible for such binding, preferably the binding of C1q and/or Fc receptors will no longer occur because of the modification of glycosylation, not because of deletion of amino acids responsible for binding.

In one instance, the antibody which is employed is not an antibody fragment.

An antibody may be prepared, expressed, created or isolated by recombinant means, such as (a) antibodies isolated from an animal (e.g., a mouse) that is transgenic or transchromosomal for the immunoglobulin genes of interest or a hybridoma prepared therefrom, (b) antibodies isolated from a host cell transformed to express the antibody of interest, e.g., from a transfectoma, (c) antibodies isolated from a recombinant, combinatorial antibody library, and (d) antibodies prepared, expressed, created or isolated by any other means that involve splicing of immunoglobulin gene sequences to other DNA sequences.

An antibody may be a human antibody or a humanised antibody. The term "human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies may include amino acid residues not encoded by human germline immunoglobulin sequences (e.g., mutations introduced by random or site-specific mutagenesis *in vitro* or by somatic mutation *in vivo).* However, the term "human antibody", as used herein, is not intended to include antibodies in which CDR sequences derived from the germline of another mammalian species, such as a mouse, have been grafted onto human framework sequences.

Such a human antibody may be a human monoclonal antibody. Such a human monoclonal antibody may be produced by a hybridoma which includes a B cell obtained from a transgenic nonhuman animal, e.g., a transgenic mouse, having a genome comprising a human heavy chain transgene and a light chain transgene fused to an immortalized cell.

Human antibodies may be prepared by *in vitro* immunisation of human lymphocytes followed by transformation of the lymphocytes with Epstein-Barr virus.

The term "human antibody derivatives" refers to any modified form of the human antibody, e.g., a conjugate of the antibody and another agent or antibody.

The term "humanized antibody" is intended to refer to antibodies in which CDR sequences derived from the germline of another mammalian species, such as a mouse, have been grafted onto human framework sequences. Additional framework region modifications may be made within the human framework sequences.

Antibodies can be tested for binding to the target protein by, for example, standard ELISA or Western blotting. An ELISA assay can also be used to screen for hybridomas that show positive reactivity with the target protein. The binding specificity of an antibody may also be determined by monitoring binding of the antibody to cells expressing the target protein, for example by flow cytometry.

Once a suitable antibody has been identified and selected, the amino acid sequence of the antibody may be identified by methods known in the art. The genes encoding the antibody can be cloned using degenerate primers. The antibody may be recombinantly produced by routine methods.

In one instance, the antibody or fragment employed has two different antibody specificities. For instance, bispecific IgG may be employed.

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. As used herein, the term "amino acid" refers to either natural and/or unnatural or synthetic amino acids, including glycine and both the D or L optical isomers, and amino acid analogs and peptidomimetics.

In some instances, a mixture of antibodies or antibody fragments may be employed. At least one of the antibodies or fragments in the mixture will have modified glycosylation and in one instance they may all do so. Any embodiment described herein with reference to an antibody or fragment may similarly be performed with a mixture of antibodies or fragments as appropriate. In one instance two, three, four, five or more different antibodies or fragments may be present in the mixture.

### Therapeutic use of antibodies

An antibody, or antibody fragment, may be used to treat or prevent any suitable or disease condition. In a particularly preferred instance, the disease or condition comprises, consists essentially of, or consists of, an inflammatory condition, particularly any mentioned herein.

Thus, the antibody or fragment may be used to suppress an inflammatory condition. Suppression may be eliminating the disease or ameliorating it. One or more symptoms of the condition may be reduced or eliminated. The inflammatory index may be reduced in the subject following application of the invention.

Thus, an antibody or antibody fragment may be used as an immunosuppressive.

Examples of conditions that an antibody or antibody fragment may be used to treat or prevent include autoimmune disease, post-operative treatment, acquired haemophilia or an alloimmune reaction. Alloimmune conditions include transplant rejection, an immunological reaction upon blood transfusion or an immunological placenta transfer condition. Examples of immunological placenta transfer conditions include haemolytic disease of the fetus and newborn (HDFN) and neonatal alloimmune thrombocytopenia (NAITP).

The inflammatory condition may, for instance, be one mediated, in whole or part, by:
(i) an antibody or antibodies;
(ii) a lymphocyte reaction;
(iii) an innate immune system reaction and/or
(iv) an alloimmune reaction and/or
(v) an autoimmune reaction

The disease or condition may be characterised by an antibody or antibodies playing a role in the development of the disease or condition.

In one instance, the antigen involved in the disease is one involved in tissue degradation involved in the disease. For instance, the antigen may be a cartilage component whose degradation is seen in arthritis.

The disease or condition may be one that is not mediated by an antibody and an administered antibody applies a general immunosuppressive effect to eliminate or reduce an unwanted immune response.

The disease or condition the antibody, or fragment, is administered to treat may be one referred to in Table 1. The antigen targeted may, in a further preferred instance, be one referred to in Table 1 below.

The antibody or fragment may be a therapeutic antibody which has been used in normally glycosylated form previously, but not in a form with modified glycosylaion. The fact that the antibody has modified glycosylation may increase the potency of the antibody in comparison to the normally glycosylated form. Alternatively, or additionally, it may reduce a side-effect seen with the glycosylated form. These effects may also be mediated by the effect of the antibody on the immune system, thus the various aspects of the invention are linked by the modification of glycosylation altering the effect of the antibody on the immune system, either in terms of suppressing a pre-existing disease or condition, in terms of increasing potency or in terms of reducing or eliminating a side effect.

The therapeutic antibodies may be against any suitable therapeutic target. For instance, the antibody may be one against a cancer antigen, an autoantigen, an antigen associated with allergy, an antigen from a pathogen (for instance from a pathogenic bacteria, virus or fungus) or from a parasite. The antibody may be one against a mediator of such conditions, for instance against a molecule important in the development of autoimmune conditions or allergy. For instance, the antibody may be one against a cytokine or an interleukin. The antibody or fragment may be one against a TNF, such as TNF-alpha.

Examples of possible targets include, but are not limited to, C5, CBL, CD147, IL-8, VLA-4, CD11a, CD18, VEGF, anti-Id, ICAM-1, CD2, EGFR, TGF-beta 2, TNF-alpha, Her2/neu, F gp, CD11/18, CD14, ICAM-3, CD80, CD4, CD23, beta2-integrin, alpha4beta7, CD52, HLA DR, CD22, CD64 (FcR), TCR alpha beta, Hep B, CA 125, EpCAM, gp120, gpIIbIIIa, CD20, IL-4, IL-5, IL-6, CD33, cadherin 11 and VNRintegrin. In one instance, the antibody or antibody fragment may be one against at least one of CD3, CD20, GPIIb/IIIa, TNF, CD25, EGF-R, CD25, Her2/neu, RSV, CD33, CD52, CD11a, IL-6R and VLA4. The antibody may be one against GM-CSF.

A number of antibodies available in glycosylated form, may, in light of the invention now be provided with modified glycosylation in order to reduce or eliminated unwanted effects of the antibodies. Possible antibodies that the invention may be applied to include, but is not limited to, 5G1.1, 5G1.1-SC, ABX-CBL, ABX-IL8, AD-159, AD-439, Antegren, Anti-CD11a, Anti-CD18, Anti-LFA1, Anti-VEGF, Antova, BEC2, BIRR-1, BTI-322, C225, CAT-152, CDP571, CDP850, Corsevin M, D2E7, Herceptin, HNK20, Hu23F2G, IC14, ICM3, IDEC-114 and IDEC-131. A further list of possible antibodies which may be provided in a form with modified glycosylation include Rituxan/MabThera (rituximab), Remicade (infliximab), Avastin (bevacizumab), Herceptin (trastuzumab), Synagis (palivizumab), Erbitux (cetuximab), Humira (adalimumab), Simponi (golimumab) and Actemra (tocilizumab). Additional antibodies that the invention may be applied to include Orthoclone, Zevalin, Bexxar, ReoPro, Simulect, zenapax, Mylotarg, Campath, Xolair, Raptiva, Avastin, Actemra and Tysabri.

**TABLE 1**

| **DISEASE** | **AUTOANTIGENS** |
|---|---|
| Addison's disease | Steroid 21-hydroxylase, 17 alpha-Hydroxylase (17OH) and side-chain-cleavage enzyme (P450scc), Thyroperoxidase, thyroglobulin and H+/K(+)- |
| Anti-GBM glomerulonephritis (related to Goodpasteur) | Anti-glomerular basement membrane (anti-GBM): noncollagenous (NC1) domains of the alpha3alpha4alpha5(IV) collagen |
| Anti-neutrophil cytoplasmic antibody-associated vasculitides (ANCA associated vasculitis)(Wegener granulomatosis, Churg-Strauss syndrome, microscopic polyangiitis) | Myeloperoxidase, proteinase 3 |
| Anti-phospholipid antibody syndrome (APS) | Negatively-charged phospholipids complexed with phospholipid binding plasma proteins (e.g. beta2GPI), cardiolipin, beta2-glycoprotein I, and (beta2GPI) |
| Autoimmune bullous skin diseases (Pemphigus). Pemphigus foliaceus (PF), fogo selvagem (FS)(endemic form), pemphigus vulgaris (PV) | IgG against keratinocytes. Specific target is desmoglein (Dsg) 1 (desmosomal Cadherins) |
| Autoimmune hemolytic anemia (AIHA) | Self-antigens on red-blood-cells |
| Autoimmune hepatitis (AIH) | Actin, antinuclear antibody (ANA), smooth muscle antibody (SMA), liver/kidney microsomal antibody (LKM-1), anti soluble liver antigen (SLA/LP) and anti-mitochondrial antibody (AMA), CYP2D6, CYP2C9-tienilic acid, UGT1A, CYP1A2, CYP2A6, CYP3A, CYP2E1, CYP11A1, CYP17 and CYP21 |
| Autoimmune neutropenia (AIN) | FcgRIIIb |
| Bullous pemphigoid (BP) | Hemidesmosomal proteins BP230 and BP180 (type XVII collagen), laminin 5, the alpha6 subunit of the integrin alpha6beta4 and p200 |
| Celiac disease | transglutaminase 2 (TG2), transglutaminase 3, actin, ganglioside, collagen, calreticulin and zonulin, thyroid, endocrine pancreas, anti-gastric and liver, anti-nuclear constituents, anti-reticulin, actin, smooth muscle, calreticulin, desmin, collagens, bone, anti-brain, ganglioside, neuronal, blood vessel |
| Chronic utricaria | Alpha-subunit of the high-affinity IgE receptor, IgE |
| Complete congenital heart block (CCHB) | Ro (Sjögens syndrome antigen A (SSA)), La (Sjögens syndrome antigen B(SSB)) |
| Diabetes type 1A (T1DM) | Islet cell autoantibodies (ICA), antibodies to insulin (IAA), glutamic acid decarboxylase (GAA or GAD), protein tyrosine phosphatase (IA2 or ICA512), Insulinoma Associated Peptide-2. The number of antibodies, rather than the individual antibody, is thought to be most predictive of progression to overt diabetes. |
| Essential mixed cryoglobulinemia | Essential mixed cryoglobulinemia antigens |
| Goodpasture's syndrome (also known as Goodpasture's disease and anti-glomerular basement membrane disease | alpha3(IV) collagen (=Goodpasture antigen) |
| Graves'disease (Basedow's disease), includes Goitre and hyperthyroidism, infiltrative exopthalmos and infiltarative dermopathy. | Thyrotropin receptor (TSHR) Thyroid peroxidase (TPO) |
| Guillain-Barré syndrome (GBS). Acute inflammatory demyelinating polyneuropathy (AIDP), acute motor axonal neuropathy (AMAN) | Gangliosides GM1, GMlb, GD1a, and GalNAc-GD1a, glycosphingolipid, myelin proteins PMP22 and P0 |
| Hemophilia - Acquired FVIII deficiency | Factor VIII |
| Idiopathic thrombocytopenic purpura (ITP) | Platelet glycoprotein (GP) IIb-IIIa and/or GPIb-IX |
| Lambert-Eaton myasthenic syndrome (LEMS) | voltage gated calcium channels |
| Mixed Connective Tissue Disease (MCTD) | IgG directed against the spliceosome, U1-snRNP |
| Multiple Myeloma | Multiple Myeloma antigens |
| Myasthenia gravis | Acetylcholine receptors (AchR), muscle-specific kinase (MuSK) |
| Myocarditis, dilated cardiomyopathy (DCM)(congestive cardiomyopathy) | heart-reactive autoantibodies against multiple antigens e.g. cardiac myosin |
| Primary biliary cirrhosis (PBC) | pyruvate dehydrogenase complex (PDC)-E2 and other members of the oxaloacid dehydrogenase family, Glycoprotein-210, p62, sp100 |
| Primary Progressive Multiple Sclerosis (PPMS) | Myelin oligodendrocyte glycoprotein (MOG),Myelin proteolipid protein (PLP), transketolase (TK), cyclic nucleotide phosphodiesterase type I (CNPase I), collapsin response mediator protein 2, tubulin beta4, neurofascin |
| Rheumatic heart disease (RHD),(Rheumatic fever) | Cardiac myosin |
| Rheumatoid Arthritis (RA) | Type II collagen, citrullin (-ated proteins (e.g. (fibrinogen, vimentin, filaggrin, type II collagen, enolase)), G6PI, RFs (anti-Fc/IgG), Vimentin, and cytokeratin |
| Sjögren Syndrome (SS) | Ro (Sjögens syndrome antigen A (SS-A)), La (Sjögens syndrome antigen B(SS-B)), p80 coilin, antinuclear antibodies, anti-thyroid, anti-centromere antibodies (Raynaud's phenomenon), anti-carbonic anhydrase II (distal renal tubular acidosis), anti-mitochondrial antibodies (liver pathology), cryoglobulins (evolution to non-Hodgkin's lymphoma). alpha- and beta-fodrin, islet cell autoantigen, poly(ADP)ribose polymerase (PARP), NuMA, Golgins, NOR-90, M3-muscarinic receptor |
| SLE including Lupus nephritis | Autoantibodies to nuclear constituents (e.g. dsDNA and nucleosomes), dsDNA, PARP, Sm, PCDA, rRNA Ribosome P proteins, C1q |
| Stiff-person syndrome (SPS) | glutamic acid decarboxylase (GAD), amphiphysin. |
| Systemic sclerosis (scleroderma) | DNA-topoisomerase I (Scl-70), U3 snRNP, U2 snRNP, 7-2 RNP, NOR-90, centromere-associated proteins, and nucleolar antigens ,Anti-Th/To, Anti-RNA polymerase I/III, Anti-PDGF receptor, Anti-fibrillin-1, M3-muscarinic receptor, |
| Transplant rejection | Transplant rejection antigens |

The therapeutic antibody may be to treat any disease or condition where antibody treatment is appropriate. The disease or condition may be any of those mentioned herein. The modification of glycosylation may result in prolonging the effect of the therapeutic antibody. It may redirect the therapeutic effect of the antibody.

The antibody or fragment may bind a red blood cell antigen. For instance, an antigen found on the surface of a red blood cell, such as a red blood cell structural protein, a blood group protein or a red blood cell carbohydrate. The antigen may be one involved in AIHA, for instance, one of the four membrane proteins that acts as an autoantigen in AIHA. The therapeutic antibody or fragment may be against an antigen involved in a haemolytic disease of the newborn or one involved in SLE

The antibody or fragment may be against a Rhesus antigen, such as the RhD, Cc and Ee antigen. Thus, the antibody may be against Rhesus D antigen, hence in a particularly preferred embodiment, the antibody is anti-D. The antibody may be one administered to treat an immune reaction by a mother against a foetus. The antibody may be one of Rhesonativ, Octapharma, Lachen, Schweiz or Diamed, but where the antibody has modified glycosylation.

Intravenous preparations of immune globulin (IVIG) are used to treat a variety of conditions. The invention may be applied to IVIG and to any disease or condition mentioned herein. Hence the invention provides IVIG where the antibodies present have modified glycosylation. Typically, IVIG is prepared using blood from a plurality of people and in one embodiment, the IVIG provided by the invention has been so provided. Conditions which may be treated using the IVIG preparations of the invention include idiopathic thrombocytopenic purpura (ITP), primary immunodeficiency, autoimmune thrombocytopenia, the vascular disorder Kawaskai disease, hematopoietic stem cell or bone marrow transplantation, particularly in patients older than 20 years, chronic B-cell lymphocytic leukemia, prevention of graft versus host disease in transplant patients, and paediatric HIV-1 infection.

IVIG preparations of the invention may also be employed to treat aplastic anemia, red blood cell aplasia, autoimmune hemolytic anemia, hemolytic disease of the newborn, patients with acquired clotting factor inhibitors, acquired von Willebrand disease, immune-mediated neutropenia (deficiency of polysegmented white blood cells), pemphigoid disorders, refractoriness to platelet transfusions, blood transfusion reactions or consequences, Graves' ophthalmopathy, pretibial myxedema, multiple sclerosis, CIDP, and various systemic autoimmune rheumatological conditions including rheumatoid arthritis, dermatomyositis and systemic lupus erythematosus (SLE). In patients at risk for infectious diseases because of compromised immune systems such as patients with burns, trauma, low birth weight or HIV infection. IVIG preparations of the invention may be used to treat any severe autoimmune disease. IVIG preparations of the invention may be used to treat carbon monoxide poisoning. IVIG may also be employed to treat Guillain-Barre syndrome, polyneuropathy, vasculitis, systemic lupus erythematosus, antiphospholipid syndrome and myaesthenia gravis. IVIG may be used to help prevent, or reduce the risk of, miscarriage. It may be used to treat hypogammaglobulinemia, pemphigus, polymyositis (PM), dermatomyositis (DM), Wegener's granulomatosis (WG), Churg-Strauss syndrome and chronic inflammatory demyelinating polyneuropathy (CIDP).

Any of the conditions mentioned herein in relation to IVIG, may also be the subject of treatment using IVIG of the invention.

The invention may result in reduction of one or more parameters forming part of an inflammatory condition. For instance, reduced Fc receptor binding may be seen. A reduction of opsonisation may occur. Reduced Fc receptor mediated phagocytosis may be seen, such as reduced Fc receptor mediated erythrophagocytosis. In some instances, reduced complement activation may be seen and a drop in activation of the oxidative burst triggered by Fc receptors may also occur. Cytokine released trigged by Fc receptor binding may also be altered or changed, for instance, IL-8 release may be reduced, particularly where the antibody is one against RBCs or IVIG is administered.

In order to assess the reduction or elimination of a particular parameter any suitable technique may be employed, including via employing the assays described in the Examples of the present application. For instance, those for assessing phagocytosis, quantification of cell bound antibody or fragment, monocyte monolayer assays (MMAs), quantification of phagocytosis of RBC via measuring RBC. ELISA, ELISPOT, chemiluminescence and Western blot based assays may be employed. Cytokine release may be measured. Complement related parameters measured, such as complement uptake, in particular C1q. In one particularly preferred embodiment, Fc receptor mediated phagocytosis of RBC will be measured, particularly where the antibody or fragment is anti-D. Any of the assays mentioned herein may be peformed *in vitro.* Assessment may be made during treatment, for instance by obtaining samples during treatment at particular timepoints and performing such assays on the samples obtained. Appropriate controls may also be used, such as a comparison with a fully glycosylated antibody. Such assessment may be performed for both where the aim is to treat or prevent a disease or condition or alternatively to reduce a side effect or increase potency.

In the case of an antibody against an RBC antigen, reduced phagocytosis and removal of RBCs by cells such as monocytes or macrophages may be seen. This may, in some instances, be evidenced by reduced hemoglobin accumulation in phagocytic cells. In one preferred instance, RBC lysis may be reduced. Such lysis may be reduced extravascularly or intravascularly or both.

Undesirable hemolysis is found as both an undesirable side effect in both administration of anti-D and also in administration IVIG. Thus, in both situations the undesirable haemolysis may be reduced and treatment improved. In one particularly preferred embodiment, therefore the invention is applied to reduce hemolysis seen with IVIG treatment. Reduced hemolysis may result in reduced intravascular coagulation.

### Modification of glycosylation

### Enzymatic modification

In one instance, the glycosylation of an antibody as disclosed herein may be enzymatically modified. Any suitable enyzme may be employed to modify glycosylation of an antibody or fragment. In one particularly preferred instance, the enzyme used for the modification is an EndoS. Thus, for any of the embodiments mentioned herein, preferably the modification to the glycosylation is that made by EndoS, or that obtainable by EndoS treatment. However, any suitable enzyme may be employed.

The EndoS polypeptide is preferably *S. pyogenes* EndoS, or a variant or fragment of *S. pyogenes* EndoS which retains immunoglobulin endoglycosidase activity. The variant may be an EndoS polypeptide from another organism, such as another bacterium. The bacterium is preferably a *Streptococcus,* such as *Streptococcus equi, Streptococcus zooepidemicus* or, preferably, *Streptococcus pyogenes.* Alternatively, the variant may be from *Corynebacterium pseudotuberculosis*, for example the CP40 protein; *Enterococcus faecalis,* for example the EndoE protein; or *Elizabethkingia meningoseptica* (formerly *Flavobacterium meningosepticum*), for example the EndoF₂ protein.

The EndoS polypeptide may, for instance, comprise:
(a) the amino acid sequence of SEQ ID NO: 1;
(b) a variant thereof having at least 50% identity to the amino acid sequence of SEQ ID NO: 1 and having immunoglobulin endoglycosidase activity; or
(c) a fragment of either thereof having immunoglobulin endoglycosidase activity.

In one preferred instance, the EndoS polypeptide comprises, or consists of, the sequence of SEQ ID NO: 1. SEQ ID NO: 1 is the sequence of the mature form of EndoS, without the signal sequence, and corresponds to amino acids 37 to 995 of SEQ ID NO: 2.

The EndoS polypeptide employed may additionally include a signal sequence. Accordingly, the EndoS polypeptide employed may comprise:
(a) the amino acid sequence of SEQ ID NO: 2;
(b) a variant thereof having at least 50% identity to the amino acid sequence of SEQ ID NO: 2 and having immunoglobulin endoglycosidase activity; or
(c) a fragment of either thereof having immunoglobulin endoglycosidase activity.

The EndoS polypeptide may consist of the sequence shown in SEQ ID NO: 2.

Variant polypeptides are those for which the amino acid sequence varies from that in SEQ ID NO: 1 or SEQ ID NO: 2, but which retain the same essential character or basic functionality as EndoS. The variant polypeptides may therefore display antibody endoglycosidase activity, particularly IgG endoglycosidase activity. Typically, polypeptides with more than about 50%, 55% or 65% identity, preferably at least 70%, at least 80%, at least 90% and particularly preferably at least 95%, at least 97% or at least 99% identity, with the amino acid sequence of SEQ ID NO: 1 or SEQ ID NO: 2 are considered variants of the protein. Such variants may include allelic variants and the deletion, modification or addition of single amino acids or groups of amino acids within the protein sequence, as long as the peptide maintains the basic functionality of EndoS. The identity of variants of SEQ ID NO: 1 or SEQ ID NO: 2 may be measured over a region of at least 100, at least 250, at least 500, at least 750, at least 800, at least 850, at least 900, at least 950, at least 995 or more contiguous amino acids of the sequence shown in SEQ ID NO: 1 or SEQ ID NO: 2, or more preferably over the full length of SEQ ID NO: 1 or SEQ ID NO: 2.

Amino acid identity may be calculated using any suitable algorithm. For example the UWGCG Package provides the BESTFIT program which can be used to calculate homology (for example used on its default settings) (Devereux et al (1984) Nucleic Acids Research 12, 387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al*, supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The variant sequences typically differ by at least 1, 2, 3, 5, 10, 20, 30, 50, 100 or more mutations (which may be substitutions, deletions or insertions of amino acids). For example, from 1 to 100, 2 to 50, 3 to 30 or 5 to 20 amino acid substitutions, deletions or insertions may be made. The modified polypeptide generally retains activity as an immunoglobulin-specific endoglycosidase. The substitutions are preferably conservative substitutions, for example according to the following Table. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Variants of the amino acid sequence of SEQ ID NO: 1 preferably contain residues 191 to 199 of SEQ ID NO: 1, i.e. Leu-191, Asp-192, Gly-193, Leu-194, Asp-195, Val-196, Asp-197, Val-198 and Glu-199 of SEQ ID NO: 1 (which correspond to residues 227 to 235 of SEQ ID NO: 2, i.e. Leu-227, Asp-228, Gly-229, Leu-230, Asp-231, Val-232, Asp-233, Val-234 and Glu-235 of SEQ ID NO: 2). These amino acids constitute a perfect chitinase family 18 active site, ending with glutamic acid. The glutamic acid in the active site of chitinases is essential for enzymatic activity. Most preferably, therefore, the variant of SEQ ID NO: 1 contains Glu-199 of SEQ ID NO: 1 and the variant of SEQ ID NO: 2 contains Glu-235 of SEQ ID NO: 2. The variant of SEQ ID NO: 1 may contain residues 191 to 199 of SEQ ID NO: 1 having one or more conservative substitutions, provided that the variant contains Glu-199 of SEQ ID NO: 1. Alternatively, the variant of SEQ ID NO: 2 may contain residues 227 to 235 of SEQ ID NO: 2 having one or more conservative substitutions, provided that the variant contains Glu-235 of SEQ ID NO: 2.

The fragment of the EndoS polypeptide used in the invention is typically at least 10, for example at least 20, 30, 40, 50 or more amino acids in length, up to 100, 200, 250, 300, 500, 750, 800, 850, 900, 950, 995 amino acids in length, as long as it retains the IgG endoglycosidase activity of EndoS. Those fragment lengths in one preferred instance are in relation to fragments from the sequence of SEQ ID No:1. In some instances, longer EndoS enzymes exist and hence longer fragments from them may be employed. For instance, a fragment may be at 1000, 1250, 1500 or 1750 amino acids in length.

Preferably, the fragment of the EndoS polypeptide used in the invention encompasses residues 191 to 199 of SEQ ID NO: 1, i.e. Leu-191, Asp-192, Gly-193, Leu-194, Asp-195, Val-196, Asp-197, Val-198 and Glu-199 of SEQ ID NO: 1 (residues 227 to 235 of SEQ ID NO: 2, i.e. Leu-227, Asp-228, Gly-229, Leu-230, Asp-231, Val-232, Asp-233, Val-234 and Glu-235 of SEQ ID NO: 2). A preferred fragment of SEQ ID NO: 2 consists of amino acids 37 to 995 of SEQ ID NO: 2, i.e. SEQ ID NO: 1, which corresponds to the form of EndoS secreted from *S. pyogenes* after removal of the signal peptide. Another preferred fragment of the invention consists of amino acids 1 to 409 of SEQ ID NO: 1 (amino acids 37 to 445 of SEQ ID NO: 2), which corresponds to the enzymatically active α-domain of EndoS generated by cleavage by the streptococcal cysteine proteinase SpeB.

The EndoS used in the invention may be chemically modified, e.g. post-translationally modified. For example, they may be glycosylated, phosphorylated or comprise modified amino acid residues. They may be modified by the addition of histidine residues to assist their purification or by the addition of a signal sequence to promote insertion into the cell membrane. Such modified polypeptides fall within the scope of the term "polypeptide" used herein.

The EndoS for use in accordance with the invention display immunoglobulin endoglycosidase activity, and in particular IgG endoglycosidase activity. Preferably, the polypeptide hydrolyzes the β-1,4-di-*N*-acetylchitobiose core of the asparagine-linked glycan of IgG. Preferably the activity may be specific for IgG. The endoglycosidase activity may be determined by means of a suitable assay. For example, a test polypeptide may be incubated with an antibody, typically IgG, at a suitable temperature, such as 37°C. The starting materials and the reaction products may then be analysed by SDS PAGE. Typically, the molecular mass of the IgG heavy chain is reduced by approximately 3kDa if the test polypeptide has endoglycosidase activity. Another assay for determining whether a test polypeptide has antibody endoglycosidase activity is by detection of glycosylated antibody using *Lens culinaris* agglutinin lectin (LCA), optionally using horseradish peroxidase and peroxidase substrate. Typically, the carbohydrate signal is reduced if the test polypeptide has antibody endoglycosidase activity. Another assay for determining whether a test polypeptide has antibody endoglycosidase activity is by incubation of a test polypeptide with purified Fc fragments followed by reduction of the sample with 10 mM dithiotreitol and mass spectroscopy (MALDI-TOF) analysis. Typically, the mass of monomeric IgG Fc is reduced by 1417 ± 14 Da if the test polypeptide has antibody endoglycosidase activity.

The endoglycosidase activity of the polypeptides can be further characterised by inhibition studies.

The endoglycosidase activity of the polypeptide may be, in a preferred instance, be IgG-specific in that the polypeptide may not degrade the other classes of Ig, namely IgM, IgA, IgD and IgE, when incubated with these immunoglobulins under conditions that permit cleavage of IgG. Where the subject is a human, the EndoS polypeptide may be capable of hydrolyzing human IgG. EndoS may be used to hydrolyze human IgG of all four subclasses (IgG₁₋₄). In preferred embodiments, the EndoS polypeptide has the ability to hydrolyze human, Rhesus monkey, mouse, rat, rabbit, horse, goat, dog and swine antibodies, particular such IgG.

Glycosyl modifying enzymes for use in the present invention may be isolated from any suitable organism that expresses an EndoS polypeptide or a variant of an EndoS polypeptide. Typically, the EndoS polypeptide is isolated from suitable EndoS expressing strains of *Streptococcus,* preferably strains of *S. pyogenes.* Suitable organisms and strains may be identified by a number of techniques. For example, *S. pyogenes* strains may initially be tested for the presence an *ndoS* gene. Polynucleotide primers or probes may be designed based on, for example, SEQ ID NOs: 1, 2 or 3. The presence of the *ndoS* gene can then be verified by PCR using such primers or by hybridisation of probes to genomic DNA of the *S. pyogenes* strain.

Streptococcal strains expressing active EndoS or a variant thereof can be identified by assaying for IgG endoglycosidase activity in the culture supernatant or by immunodetection using antibodies directed towards EndoS. The Streptococcal strains that have been verified as expressing active EndoS are the *S. pyogenes* M1 serotype strains AP1 and SF370, the *S. equi* strain 4047 and the *S. zooepidermicus* strain H70. In addition, the *ndoS* gene is found in the following *S. pyogenes* strains: M1 serotype strains SSI-1 and MGAS5005, M2 serotype strain MGAS10270, M3 serotype strain MGAS315, M4 serotype strain MGAS10750, M5 serotype strain Manfredo, M6 serotype strain MGAS10394, M12 serotype strain MGAS9429, M18 serotype strain MGAS8232, M28 serotype strain MGAS6180 and M49 serotype strain 591.

Isolation and purification of EndoS from an expressing *S. pyogenes* culture, or from cultures of other cells expressing EndoS is typically on the basis of antibody endoglycosidase activity, particularly IgG activity. Preferably the purification method involves an ammonium sulphate precipitation step and an ion exchange chromatography step. According to one method, the culture medium is fractionated by adding increasing amounts of ammonium sulphate. The amounts of ammonium sulphate may be 10 to 80%. Preferably the culture medium is fractionated with 50% ammonium sulphate, and the resulting supernatant is further precipitated with 70% ammonium sulphate. Pelleted polypeptides may then be subjected to ion exchange chromatography, for example by FPLC on a Mono Q column. Eluted fractions may be assayed for IgG endoglycosidase activity and peak activity fractions may be pooled. Fractions may be analysed by SDS PAGE. Fractions may be stored at -80°C.

In an alternative method to purify EndoS, EndoS without the signal sequence (i.e. having the sequence of SEQ ID NO: 1) is expressed in *Escherichia coli* using GST Gene Fusion System (Amersham-Pharmacia Biotech, Uppsala, Sweden). A 2929 base pair PCR product covering bases 304 to 3232 of the *ndoS* sequence is amplified from *S. pyogenes* genomic DNA using primers 5'-ACT-GGG-ATC-CCG-GAG-GAG-AAG-ACT-3' with a *Bam*HI site (underlined) and 5'-TTA-ATC-TCG-AGG-TTG-CTA-TCT-AAG-3' with an *Xho*I site (underlined). This fragment is digested with *Bam*HI and *Xho*I and ligated into the pGEX-5X-3 generating plasmid pGEX*ndoS* that is used to transform *E. coli* BL21(DE3)pLys. pGEX*ndoS*/BL21(DE3)pLys is induced with 0.1 mM isopropyl β-D-thiogalactopyranoside. After induction, bacteria are lysed using BugBuster™ (Novagen) and the GST-EndoS fusion protein is purified on Glutathione-Sepharose®. The GST tag is removed using factor Xa according to protocols (Amersham-Pharmacia Biotech), and residual factor Xa is removed using Xarrest™-agarose (Novagen). This results in a preparation of recombinant EndoS (rEndoS) that is homogenous as assessed by SDS-PAGE and Western blot using EndoS-specific antibodies. Prior to *in vivo* experiments protein samples are sterile-filtered through a 0.2 µm filter (Millipore). Purified EndoS protein is stored at -80°C in phosphate buffered saline.

EndoS enzymes for use in the invention may also be prepared as fragments of such isolated polypeptides. Further, the EndoS polypeptides may also be made synthetically or by recombinant means. For example, a recombinant EndoS polypeptide may be produced by transfecting mammalian cells in culture with an expression vector comprising a nucleotide sequence encoding the polypeptide operably linked to suitable control sequences, culturing the cells, extracting and purifying the EndoS polypeptide produced by the cells.

A number of side chain modifications are known in the art and may be made to the side chains of the EndoS polypeptides, provided that the polypeptides retain antibody endoglycosidase activity and in particular in relation to IgG.

In one instance, the modification may be performed using an enzyme in isolated form, for instance EndoS. In others the system that the antibody or fragment is being synthesised may also express a glycosyl modifying enzyme to bring about removal or modification of the antibody glycosylation. In particular, a system may be employed which expresses both the antibody or antibody fragment and Endo S. In one instance, the host cell employed may comprise nucleotide sequences that encode and can express both the antibody, or fragment, and the enzyme, in particular Endo S.

In further instances, the enzyme employed may be EndoE. In one instance, any suitable enzyme producing the same, or equivalent, modification to EndoS or EndoE may be employed, in a further instance EndoS or Endo E themselves are employed and in a preferred instance EndoS may be employed. In one instance, Endo F₂ is employed, for instance EndoF₂ from *Elizabethkingia meningoseptica.* EndoE from *Enterococcus faecalis* may be employed in one instance. A further enzyme that may be employed in the invention is N-glycosidase F (PNGase F), StrH from *Streptococcus pneumoniae* or EndoC from group C *Streptococci.*

In one preferred instance, purified antibodies are incubated with EndoS fused to Glutathione S-transferase (GST) in phosphate buffered saline for one hour at 37°C.

The amount of GST-EndoS may be in the range 1-20 µg per mg of antibodies and the final antibody concentration is typically between 0.5 and 50 mg/ml. Subsequently, GST-EndoS can if preferred be removed from the reaction mixture by passing three times through a 0.5 - 5 ml Glutathione-Sepharose column. Alternatively, antibodies can be purified from the reaction mixture by standard affinity chromatography methods.

In another instance, purified antibodies are incubated with the enzymes EndoS, EndoE, EndoC, EndoF2, or StrH in phosphate buffered saline for 1-20 h at 37°C. The amount of enzyme may typically be in the range 1-100 µg per mg of antibodies and the final antibody concentration is typically between 0.5 and 50 mg/ml. Subsequently, antibodies can be purified from the reaction mixture by standard affinity chromatography methods.

In one instance, the enzyme used has been processed by the cysteine proteinase SpeB, particularly where the enzyme is EndoS.

### Other modification methods

Also disclosed are methods to modify the glycosylation of an antibody relative to normal glycosylation by starting with an aglycosylated antibody and treating it with appropriate transferase enzymes, for example a GlcNAc-transferase enzyme, in order to produce the desired modified glycosyl group. Only a single GlcNAc group may be added. A fucose may also be added.

Alternatively, the glycosylation of an antibody may be modified *de novo* by modification of an antibody producing cell-line (e.g. CHO, NSO, Sp2/0, Pichia pastoris, Lemna) such that it expresses the enzymes needed to produce an antibody with the desired modified glycosyl group. For example, the cell line may be modified to knock out mannose transferase enzymes such that only GlcNAc units may be added, alternatively the cell line may be transfected with only an appropriate GlcNAc-transferase.

### Diseases and Conditions

Also disclosed are antibodies or antibody fragments which may be used to treat or prevent any suitable disease or condition, particularly one that comprises, consists essentially of, or consists of, an inflammatory condition.

The disease or condition may be one mediated, in whole or part, by, or in which is present, one or more of the following:
(i) an antibody or antibodies;
(ii) a lymphocyte reaction;
(iii) an innate immune system reaction and/or
(iv) an alloimmune reaction.

In some cases, at least one of (i) to (iv) may be responsible for inflammation and/or damage seen in a condition. The condition or disease may be one where antibodies are responsible for damage, such as, for instance, tissue damage.

The disease may be a disease or condition mediated by an antibody or antibodies, particularly by an IgG antibody. It is well known in the art that antibodies are involved in the pathogenesis of a number of different diseases and conditions. The role of pathogenic antibodies in such diseases can be inhibited using an antibody or antibody fragment with modified glycosylation, even where the antibody or fragment recognises a different epitope to the disease mediating antibody.

For instance, examples of disease include autoimmune disorders, allergies, cardiovascular disease, cancer, infectious disease, and metabolic disease. In some instances, the condition may be where the subject has received a transplant, for instance to reduce or eliminate side effects of drug treatment given after transplantation or to reduce an immune reaction against the transplanted organ. The condition may, in some instances, be post-inflammatory side effects due to surgery.

The disease or condition can be an autoimmune disease. Such diseases include Addison's disease, alopecia areata, ankylosing spondilitis, antiphospholipid syndrome, aplastic anaemia, autoimmune gastritis, autoimmune hearing loss, autoimmune haemolytic anaemias, autoimmune hepatitis, autoimmune hypoparathyroidism, autoimmune hypophysitis, autoimmune inner ear disease, autoimmune lymphoproliferative syndrome, autoimmune myocarditis, autoimmune oophoritis, autoimmune orchitis, autoimmune polyendocrinopathy, Beçhet's disease, bullous pemphigoid, cardiomyopathy, chronic inflammatory demyelinating polyneuropathy, Churg-Strauss syndrome, coeliac disease, Crohn's disease, CREST syndrome, Degos disease, epidermolysis bullosa acquisita, essential mixed cryoglobulinaemia, giant cells arteritis, glomerulonephritis, Goodpasture's syndrome, Graves' disease, Guillan-Barre syndrome, Hashimoto's thyroiditis, idiopathic thrombocytopenic purpura, inflammatory bowel disease, Kawasaki's disease, Meniere's syndrome, mixed connective tissue disease, Mooren's ulcer, multiple sclerosis, myasthenia gravis, pemphigus foliaceous, pemphigus vulgaris, pernicious anaemia, polyarteritis nodosa, polyglandular autoimmune syndrome type 1 (PAS-1), polyglandular autoimmune syndrome type 2 (PAS-2), polyglandular autoimmune syndrome type 3 (PAS-3), polymyositis/dermatomyositis, primary biliary cirrhosis, psoriasis, psoriatic arthritis, Raynaud's syndrome, Reiter's syndrome, rheumatoid arthritis, sarcoidosis, scleroderma, Sjögren's syndrome, subacute thyroiditis, sympathetic opthalmia, systemic lupus erythematosus, Takayasu's arteritis, type 1 diabetes mellitus, vitiligo, Vogt-Koyanagi-Harada disease or Wegener's granulomatosis. Preferably the autoimmune disease is rheumatoid arthritis (RA), systemic lupus erythematosus or idiopathic thrombocytopenic purpura.

The disease to be treated may be an autoimmune disease and in particular one involving red blood cells, preferred examples of which include autoimmune hemolytic anaemia (AIHA), hemolytic diseases of the newborn, and Systemic Lupus Erythematosus. The disease or condition can be asthma. The asthma can be acute or chronic asthma.

The disease or condition may be an immunological placenta transfer condition, particularly one selected from haemolytic disease of the fetus and newborn (HDFN) and neonatal alloimmune thrombocytopenia (NAITP).

The disease or condition may involve unwanted activation of the classical pathway of the complement system. The antibody or antibody fragment can therefore be used to treat diseases and conditions where complement activation is detrimental to the patient. For example, they can be used to treat transplantation-derived disorders, for example transplant rejection (such as acute or chronic allograft or xenograft rejection) and graft-versus-host disease. The transplantation-derived disorder may occur due to the transplantation of a tissue or an organ in a patient.

The antibody or antibody fragment are also of use in post-operative treatment, for example in the treatment of patients who have undergone heart by-pass operations. Further, the antibody or fragment can be used for the treatment of acquired haemophilia, i.e haemophiliac patients who have developed autoantibodies against coagulation factors.

In one preferred instance, the disease to be treated is selected from the group consisting of rheumatoid arthritis, immune thrombocytopenic purpura (ITP), Systemic Lupus Erythematosus (SLE), myasthenia goodpasteures and Sj grens syndrome. In a particularly preferred embodiment, the disease to be treated is arthritis, particularly rheumatoid arthritis.

The subject is typically a mammalian subject, such as a mouse, rat or primate (e.g. a marmoset or monkey). The subject may be human or a non-human animal. The subject may be an agricultural or sports animal, for example a bovine, porcine or ovine animal, such as a cow, pig or sheep, or a horse. The animal may be a pet, such as a dog or cat.

Where the subject is a laboratory animal such as a mouse, rat or primate, the animal may be treated to induce a disease or condition mediated by pathogenic antibodies. For example, the mouse anti-CII antibody induced arthritis (CAIA) model described by Nandakumar et al. (Am. J. Pathol. 163(5): 1827-1837, 2003), or a modified version of that model, may be used.

The subject may be one who has not been administered a enzyme capable of modifying antibody glycosylation, for instance any of those mentioned herein and in particular EndoS. The treatment to be administered preferably does not involve administration also of an endoglycosylase. The subject may not have been administered EndoS and EndoS is not administered as part of the therapy. The antibody or fragment administered to the subject may not originate from the subject themselves.

The antibody or fragment may be employed in relation to any disease or condition mentioned herein in relation to suppressing an immune condition, eliminating or reducing an undesired immune system effect associated with an antibody, or increasing potency of such a therapeutic antibody.

### Therapy and Prophylaxis

Disclosed herein is the use of an antibody or antibody fragment with modified glycosylation to treat or prevent a disease or condition comprising an inflammatory condition. In one instance, one mediated by pathogenic antibodies, particularly IgG. Treatment may be therapeutic or prophylactic. The antibodies or fragments may take advantage of the effect of modification of glycosylation to also increase the efficacy of therapeutic antibodies. Thus, there may be increased potency of therapeutic antibodies and/or the prevention of unwanted side-effects of therapeutic antibodies and the elimination of unwanted immune system effects triggered by an antibody or fragment when administered as a therapeutic agent.

The antibody or fragment may be administered to an individual in order to prevent the onset of one or more symptoms of the disease or condition. In this embodiment, the subject may be asymptomatic. The subject may have a genetic predisposition to the disease. A prophylactically effective amount of the antibody or fragment is administered to such an individual. A prophylactically effective amount is an amount which prevents the onset of one or more symptoms of a disease or condition.

A therapeutically effective amount of an antibody or fragment is an amount effective to ameliorate one or more symptoms of a disease or condition. Preferably, the individual to be treated is human.

An antibody or fragment may be administered to the subject by any suitable means. The antibody or fragment may be administered by enteral or parenteral routes such as via oral, buccal, anal, pulmonary, intravenous, intra-arterial, intramuscular, intraperitoneal, intraarticular, topical or other appropriate administration routes. In one particularly preferred instance, the administration of the antibody or fragment of the invention is intravenous or subcutaneous.

The antibody or fragment may be administered to the subject in such a way as to target therapy to a particular site. For example, an antibody or fragment may be administered directly to the site of a transplanted organ. The antibody or fragment may be injected locally, for example intra-articularly or in one or more joints, particularly a joint or joints affected by arthritis. Local administration of antibody or fragment to the joints is particularly preferable for the prophylaxis or treatment of rheumatoid arthritis (RA). The antibody or fragment polypeptide may be conjugated with reagents that bind cartilage specifically.

The formulation of any of the antibody or fragment mentioned herein will depend upon factors such as the nature of the antibody or fragment and the condition to be treated. The antibody or fragment may be administered in a variety of dosage forms. It may be administered orally (e.g. as tablets, troches, lozenges, aqueous or oily suspensions, dispersible powders or granules), parenterally, subcutaneously, intravenously, intramuscularly, intrasternally, transdermally or by infusion techniques. The antibody or fragment may also be administered as suppositories. A physician will be able to determine the required route of administration for each particular patient.

Typically the antibody or fragment is formulated for use with a pharmaceutically acceptable carrier or diluent and this may be carried out using routine methods in the pharmaceutical art. The pharmaceutical carrier or diluent may be, for example, an isotonic solution. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for intravenous or infusions may contain as carrier, for example, sterile water or preferably they may be in the form of sterile, aqueous, isotonic saline solutions.

For suppositories, traditional binders and carriers may include, for example, polyalkylene glycols or triglycerides; such suppositories may be formed from mixtures containing the active ingredient in the range of 0.5% to 10%, preferably 1% to 2%.

Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the pharmaceutical composition is lyophilised, the lyophilised material may be reconstituted prior to administration, e.g. a suspension. Reconstitution is preferably effected in buffer.

Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

Pharmaceutical compositions suitable for delivery by needleless injection, for example, transdermally, may also be used.

A therapeutically effective amount of an antibody or fragment is administered. The dose may be determined according to various parameters, especially according to the polypeptide or polynucleotide used; the age, weight and condition of the patient to be treated; the route of administration; and the required regimen. Again, a physician will be able to determine the required route of administration and dosage for any particular patient. A typical daily dose is from about 0.1 to 50mg per kg, preferably from about 0.1mg/kg to 10mg/kg of body weight, according to the activity of the specific inhibitor, the age, weight and conditions of the subject to be treated, the type and severity of the disease and the frequency and route of administration. Preferably, daily dosage levels are from 5mg to 2g.

In some instances, more than one antibody or fragment with modified glycosylation may be administered to the subject. For instance, five, four, three or two different monoclonal antibodies or fragments with modified glycosylation may be administered. In some cases, five or less, preferably four or less and more preferably three or less different monoclonal antibodies or fragments with modified glycosylation may be administered.

In one preferred instance, the treatment will involve administration of the antibody or fragment direct to the subject and preferably the treatment will not involve removal of antibodies from the subject and modification of glycosylation of them before readministration. Thus, the treatment may lack an *ex vivo* step. The treatment may not involve returning EndoS treated blood to the subject. In the present invention, what is administered is polyclonal, that is IVIG. What is administered may be an antibody or fragment which does not originate from the subject.

The subject may display, in some instances, reduced complement activation, phagocytosis, opsonisation (particularly by a therapeutic antibody), inflammatory mediator release (such as cytokine release, particularly interleukin release, especially IL-8 release), respiratory burst activation and/or degranulation. The subject may display reduced fever, swelling or other symptoms resulting from an inflammatory condition. Treatment may result in improved mobility. The subject may display reduced transplant rejection. In the instance of the administration of a therapeutic antibody, preferably the modification of full glycosylation will not prevent the therapeutic effect, thus at least some, preferably the majority and particularly preferably all of the therapeutic effect will be retained in comparison to the glycosylated form of the antibody, whilst the unlimited side effects will be reduced or eliminated. In a particularly preferred instance, hemolysis will be reduced.

Parameters which may be modulated using the invention may, in some instances, include antibody-dependent cellular cytotoxicity (ADCC), antibody-dependent cell-mediated phagocytosis (ADCP), complement-dependent cytotoxicity (CDC), CIq binding, FcR binding (half-life), protein A binding, and/orprotein G binding.

### Examples

### Example 1 - Monoclonal antibodies with modified glycosylation dominantly suppress the induction of arthritis

Collagen antigen induced arthritis (CAIA) was used to assess the effect of antibodies with modified glycosylation on arthritis. Groups of six months old male QB = (BALB/c x B10.Q) F1 mice were injected intravenously with 9 mg of the two arthritogenic mAbs cocktail (M2=M2139 and C1=CIIC1) with or without EndoS treated (modified glycosylation) mAbs (M2D=M2139 modified glycosylation and C1D = CIIC1 with modified glycosylation) at different concentrations on day 0.

In particular, the following concentrations of monoclonal antibodies were administered:
(i) M2 (4.5 mg) + C1 (4.5 mg);
(ii) M2D (4.5 mg) + C1D (4.5 mg);
(iii) M2 (3.375 mg) + C1 (3.375 mg) + M2D (1.125 mg) + C1D (1.125 mg); and
(iii) M2 (3.9375 mg) + C1 (3.9375 mg) + M2D (0.5625 mg) + C1D (0.5625 mg).

On day 5, all the mice received 25 µg of lipopolysaccharide (LPS) from *E.coli 055:B5* administered intraperitoneally. Mice were monitored for arthritis development.

Figure 1, shows arthritis incidence (A) and mean arthritis score (B) of arthritic animals only are shown. As can be seen, arthritis was only induced when the two monoclonal antibodies were administered in normally glycosylated form. The modified glycosylated forms of the antibodies were incapable of inducing arthritis, themselves.

Unexpectedly, not only were the modified glycosylated forms incapable of inducing arthritis, they acted dominantly, suppressing the induction of arthritis by the glycosylated forms of the antibodies at all concentrations tested, even where the disease causing glycosylated forms were present at far higher concentrations than the modified glycosylated forms.

### Example 2 - Joint Histology of CAIA mice

Joint histopathology was performed on the ankle joints of (BALB/c x B10.Q) F1 mice (n=3-4) injected with 9 mg of (i) untreated M2 and C1 monoclonal antibodies; (ii) modified glycosylation forms of the two antibodies generated using EndoS; and (iii) an equal mixture of both untreated and EndoS modified antibodies.

Ten-fold magnification showed a massive infiltration of immune cells, fibrin deposition, cartilage and bone-erosions in the mice of (i), but only very mild bone erosion in modified glycosylated mAbs treated mice. Thus, again, not only did the modified antibodies fail to induce arthritis, but they were also capable of the dominant suppression of arthritis by the glycosylated monoclonal antibodies.

### Example 3 - Antibodies with modified glycosylation are capable of dominantly suppressing the induction of arthritis by monoclonal antibodies against different epitopes

Groups of two months old male QB = (BALB/c x B10.Q)F1 mice were injected intravenously with 4 mg of the four arthritogenic mAbs cocktail (M2=M2139; C1=CIIC1; C2=CIIC2 and UL1) with or without EndoS treated (modified glycosylation) mAbs (M2D=M2139 modified glycosylation and C1D = CIIC1 modified glycosylation) on day 0. On day 5, all the mice received 25 µg per mouse of lipopolysaccharide (LPS) from *E.coli 055:B5* intraperitoneally. Mice were monitored for arthritis development.

Figure 1 shows arthritis incidence (panel C) and mean arthritis score (panel D) of arthritic animals only, with n indicating the number of mice used in each group. Error bars indicate ± SEM. As can be seen from the results depicted, even though the antibodies with modified glycosylation against M2 and C1 recognise different epitopes to the glycosylated monoclonal antibodies against C2 and UL1, they were still unexpectedly able to dominantly suppress the induction of arthritis in the mice.

### Example 4 - Inhibition of autoantibody destruction of red blood cells

The effect of modification of glycosylation of anti-D antibodies was studied as a model to demonstrate the effect of modifying the glycosylation of antibodies already used therapeutically

### Material and methods

### Protein and reagents

Blood was collected from healthy donors. Plasma from patients with available clinical and laboratory information for these cases was obtained from the Haematology Department and Blood Centre at the University Hospital in Lund. Approval was obtained from the institutional review board for these studies. Full-length EndoS with glutathione-S-transferase (GST) as a fusion was recombinantly expressed and purified from *Escherichia coli* harboring the plasmid pGEX*ndoS* (Collin et al (2001) Infect Immun., 69:7187-7189). RPMI 1640 medium and Hank's balanced salt solution (HBSS) were from GIBCO, Paisley, U.K. All other reagents were purchased from Sigma-Aldrich, Sweden AB, unless indicated otherwise.

### RBC preparation and sensitization

Blood, drawn from healthy Rh-positive individuals was collected in heparin containing tubes. After centrifugation at 1100 x g for 10 minutes, plasma and buffy coat-aspirated and red blood cells were washed 5 times with 10 volumes of phosphate buffered saline (PBS): 10 mM phosphate buffer, pH 7.4, 120 mM NaCl, 3 mM KCl. RBC, 1 ml of 5% suspension, were incubated with 100 :1 commercial human anti-D antibody (Rhesonativ™, Octapharma™, Lachen™, Schweiz™ or Diamed™, BIO-RAD, Hercules, CA, USA) at 37°C for 1 hour. RBC were then washed five times and subjected to phagocytosis assay. Sensitized RBC were assayed by ELISA using peroxidase-conjugated rabbit-anti human IgG or goat-anti rabbit IgG (BIO-RAD).

### SDS-PAGE and Western blot analysis

Sodium dodecyl sulfate-polyacrylamide gel electrophoresis (SDS-PAGE) was performed as previously described using Mini Protean II cell equipment from BIORAD (Hercules, CA, USA) and the buffer system described by Laemmli. (Laemmli et al (1970) Nature, 227: 680-685). For immunoblotting, the gels were transferred to polyvinylidenefluoride (PVDF) membranes (Immobilon P, Millipore, Bedford, MA) as described by Matsudaira (Matsudaira et al (1987) J Biol Chem., 262: 10035-10038). After blotting, membranes were blocked in PBS supplemented with 0.05% v/v Tween 20 (PBST) and 5% w/v skim milk (DIFCO™, Detroit, MI) for 20 minutes at room temperature.

For detection of IgG, the blots were subsequently washed in PBST and then incubated with peroxidase-conjugated anti-human IgG or HRP anti-rabbit IgG (diluted 1:2000) (BIO-RAD, Hercules, CA) for one hour at 37°C. The membranes were developed using SuperSignal West Pico (PIERCE, Rockford, IL) according to the manufacturer's instructions before analyzing by the Chemidoc XRS imaging system and Quantity One image analysis software (BIO-RAD).

### Phagocytosis assay

RBC sensitized with anti-D, 100-500 :1 of 5% solution were added to 2-10 x 10⁶ blood monocytes or THP-1 cells in PBS and incubated 60 min at 37°C in suspension with gentle agitation. Nonadherent RBC were removed by five washes with PBS. Noningested RBC were lysed with 0.5 ml distilled water for 20 second and followed with two washes with PBS. This treatment has been described to entirely lyse adherent RBC without influencing the integrity of monocytes (Bussolino et al (1987) Br J Haematol., 66:271-274).

### Quantification of RBC-bound IgG

RBC were sensitized with human anti-D or rabbit anti-human RBC (see above) with following washes in PBS. After that cells were pelleted by centrifugation at 1000 x g for five minutes, resuspended in lysis buffer containing 20 mM Tris-HCl pH 7.4, 0.150 M NaCl, 1% v/v Triton-100 and 0.25% v/v NP40 for ten minutes at 4°C. Next, the samples were centrifuged for ten minutes at 14000 x g and supernatants analyzed for IgG by polyacrylamide gel/ immunoblotting and ELISA.

### FITC-labelling of RBC

Erythrocyte-labelling with fluorescein isothiocyanate (FITC) was performed according to the earlier description (Tippett et al (2007) J Immunol Methods., 325:42-50). Briefly, RBC were resuspended to 1 x 10⁸/ml in fresh 0.5 M sodium bicarbonate, pH 9.0 and labelled with, 200 :g/ml-500 :g/ml (FITC) (PIERCE™, Rockford, IL) for 30 minutes at room temperature with constant agitation following 5 washes in cold PBS. After that FITC-labelled RBC were sensitized with anti-D as described above.

### Monocyte monolayer assay

The MMA was performed as previously described (Nance et al (1987) Transfusion, 27:449-452). Mononuclear cells were separated from heparinised human whole blood from healthy volunteers using the Polymorphprep preparation kit (AXIS-SHIELD™, Oslo, Norway) or Ficoll-Paque Plus (Amersham Biosciences, Uppsala, Sweden) according to instructions provided by the manufacturers. After isolation, the mononuclear cells were washed 3 times with PBS and finally resuspended in RPMI-medium containg 10% fetal calf serum to the concentration 4 to 6 x 10⁶ cells/ml. Monocyte monolayers were prepared by incubating 0.2 ml of mononuclear cells in Lab-Tek Chamber Slides (Nunc, A/S, Roskilde, Denmark) at 37°C for 1 hour. Nonadherent cells and RPMI were removed by aspiration with a pipette. Sensitized RBC, 0.2 ml or control RBC resuspended in RPMI containing 10% fetal calf serum were then added to the mono layer and incubated for 1 h at 37°C.

The slides were then rinsed gently with PBS to remove nonadherent red cells. Dried slides were stained with May Grünvald-Giemsa. Monocytes were examinated microscopically, and the frequency of cells with adherent or phagcytosed red cells was estimated.

### Quantification of haemoglobin

Haemoglobin quantification was performed spectrophotometrically at 541 nm or by using the chromogen 2,7 diaminofluorene at 630 nm. Briefly, a working solution was prepared by adding 1 volume of 1% w/v 2,7 diaminofluorene in 90% glacial acetic acid to 10 volumes of 0.2 M Tris-HCl, pH 7.2 and 0.02 volumes of 30% hydrogen peroxide. 100 microliters of working solution was added to 10-100 :1 of RBC supernatants. After 20 min of incubation at room temperature, the optical density at 600 nm was measured in Wallac 1420 Multilabel Counter (PerkinElmer, Turku, Finland).

### Cell preparations

The human monocytic cell line THP-1 (ATCC, Rockville, Md) was cultured in RPMI 1640 medium supplemented with 10% fetal calf serum and 10 µg/ml gentamycin at 37°C in an atmosphere containing 5% CO₂ and 95% humidity. Nunclon flasks for cell culture were used (Nunc). Monocytes were isolated from human whole blood using the Polymorphprep preparation kit (AXIS-SHIELD™, Oslo, Norway) or Ficoll-Paque Plus™ (Amersham Biosciences, Uppsala, Sweden) according to instructions provided by the manufacturers. After isolation, the cells were counted and resuspended in PBS or RPMI-medium with 10% calf serum.

### Enzyme linked immunosorbent assay (ELISA)

For glycan detection, microtiter plates (NUNC, Roskilde, Denmark) were coated with 100 :1 of solubilised RBC diluted 10 times in PBS and kept at 4°C overnight. The plates were washed three times with lectin buffer containing 10 mM HEPES, pH 7.5, 0.15 M NaCl, 0.01 mM MnCl₂, 0.1 mM CaCl₂ and 0.1% v/v Tween 20 and blocked in the same buffer for one hour at room temperature.

In the next step 1 µg/ml biotinylated LCA-lectin was added and incubation continued for 1 hour at 37°C. After three washes with lectin buffer, 1 µg/ml biotinylated *Lens culinaris* agglutinin (LCA)-lectin (Vector Laboratories, Burlingame, CA, USA) was added and incubation continued for 1 hour at 37°C. Following three more washes, 0.1µg/ml peroxidase labeled streptavidin (Vector Laboratories) was added and the plate was incubated for 1 hour at 37°C.

The colour reaction was developed using tetramethylbenzidine/hydrogen peroxide containing substrate solution (R&D Systems Europe, Ltd. UK). The absorbance was read on a model Wallac 1420 Multilabel Counter (PerkinElmer, Turku, Finland) at 450 nm.

For detection of RBC-bound IgG the plate was coated with solubilized RBC diluted 10 times in PBS for 20 hours at 4°C. Next day, the plate was blocked with PBS supplemented with 0.05% v/v Tween 20 (PBST) and 2% w/v bovine serum albumin for 2 hours at room temperature. After this step, a peroxidase-antihuman IgG or peroxidase-conjugated antirabbit IgG (dilution 1:1000) (BIO-RAD, Hercules, CA, USA) was used for detection. The plate was washed three times with PBST after the coating step and between each of the following incubation steps. The colour reaction was performed as above.

### IL-8 assay

Human IL-8 was detected using DuoSet™ ELISA R&D Systems, Inc. MN, USA. Experiments were performed using 1 x 10⁶ cells per ml in 12-well cell culture plates (NUNC™, Roskilde, Denmark). Briefly, THP-1 cells or monocytes purified from peripheral blood were incubated with 0.5-1% suspension of anti-D sensitized RBC or control RBC (unsensitized) for two hours (blood monocytes) and 24 hours (THP-1 cells) at 37° C in humidified air. The supernatants were analyzed for IL-8. Relative means and standard errors indicated with error bars were calculated from three experiments.

### C1q uptake studies

C1q binding to sensitized RBC were analyzed as described previously (Basta et al (1991) Blood, 78:700-702). Briefly, blood was collected into EDTA-tube. RBC 3.3 x 108 cells/ml in isotonic Veronalbuffered saline containing 0.1% gelatin, 0.15 mM CaCl₂ and 1 mM MgCl₂ (GVBS2+). Three hundred microliters RBC were incubated with equal volume of rabbit IgG antihuman RBC, with or without treatment with EndoS, or buffer for 30 minutes at 37°C. After two washes in GVBS2+ RBC pellets were resuspended in 250 :1 human serum and placed in 30°C for 15 minutes. The cell pellets were then washed and flourescein-conjugated anti-human C1q (Cappel, MP Biomedicals, LLC Solon, Ohio) added for 60 minutes incubation. After three washes of cell pellets fluorescence intensity was measured using a Wallac 1420 Multilabel Counter.

### Chemiluminescence test (CLT)

Purified peripheral blood monocytes were washed four times in PBS containing 0.2% w/v bovine serum albumin and finally resuspended to 2 x 10⁶ cells/ml in Hanks balanced salt solution (HBSS) containing 30% RPMI medium and 3% fetal calf serum (v/v). The cells were pipetted into 96 well microplate and incubated for 2 hours at 37°C in 5% CO₂. RBC, 50 :1 of 5% suspension in PBS, 0.5% human serum albumin (were sensitized with 50 :1 plasma from anti-D positive donors on 96 V-well plate for 1 hour at 37°C. After repeated washes of plate with PBS, sensitized RBC were incubated with EndoS 10-20 :g EndoS for 30 min-1 h at 37°C resupended in HBSS. In some cases plasma was pre-treated with EndoS before sensitization of RBC. Luminol, 0.8 mM, and RBC, 20 :1 were then added to wells containing monocytes.

Chemiluminescence responses were monitored for 1 hour with 5 minutes intervals using a luminometer Wallac 1420 Multilabel Counter. Monocytes responses to sensitized RBC were compared to unsensitized control RBC and expressed as a ratio (opsonic index).

### Statistics

Student *t*-test were performed to determine statistical significance designated as follows: *, *P*<0.05; **, *P*<0.01; and ***, *P*<0.001.

### Results

The results obtained demonstrate that the use of antibodies with modified glycosylation is able to change the interaction of the antibody with the immune system, increasing potency and reducing side-effects.

### EndoS inhibits phagocytosis of sensitized red blood cells by monocytes

The effects of modification of glycosylation by EndoS on IgG/Fc gamma receptor interactions was measured using autoantibody-mediated lysis of RBC, including phagocytosis via Fc gamma receptors and hemolysis, as an experimental model. For this purpose RBC were incubated with human IgG antibodies against Rhesus D antigen (anti-D), with or without preincubation of antibody with EndoS. The amount of cell-bound IgG was determined by Western immunoblot and ELISA subsequent to solubilization of sensitized RBC and IgG purification.

The results obtained show that the treatment of anti-D with EndoS does not affect the antigen recognition by this antibody and the amount of RBC-bound IgG was not influenced by EndoS treatment (see Figures 2A and 2B). Thus, modification of glycosylation does not prevent the anti-D being therapeutically effective.

EndoS hydrolysis of the N-glycan on IgG was confirmed by Western immunoblot showing a characteristic reduction in the size of IgG and decreased reactivity of EndoS-treated anti-D with *Lens culinaris agglutinin* (LCA) lectin using ELISA (Figures 2A and 2C).

The effect(s) of modification of glycosylation on phagocytosis of anti-D IgG sensitized RBC by monocytes was analyzed using peripheral blood monocyte monolayer assay (MMA). Monolayers of monocytes purified from blood were cultured on slides and anti-D sensitized RBC were added to the mono layers and incubated. The degree of phagocytosis was determined using microscopy and expressed as amount of monocytes with adherent/phagocytosed RBC by analyzing 100 monocytes. As expected the phagocytosis of IgG-sensitized RBC was significantly greater than non-sensitized RBC and was efficiently eliminated by pre-treatment of anti-D with EndoS to achieve modification of glycosylation (Figure 3A). Generally, the number of "rosettes" (three or more RBC attached to each monocyte) was increased for anti-D sensitized RBC but not for RBC sensitized with anti-D pre-treated with EndoS (Figure 3B).

To establish the extent of the intracellular ingestion of sensitized RBC, with and without treatment of anti-D with EndoS to bring about modification of glycosylation, RBC were incubated with blood monocytes or THP-1 cells, grown in suspension. Extracellularly bound RBC were lysed with distilled water (a method that does not influence the intracellular environment of monocytes) and the amount of ingested RBC determined by measuring the concentration of internalized hemoglobin using 2,7-diaminoflourene (DAF), a reagent that is specifically oxidized by haemoglobin. This experiment showed a huge increase in accumulation of intracellular haemoglobin, explained by a phagocytosis of opsonized RBC, contrary to efficiently abolished phagocytosis of RBC sensitized with EndoS pre-treated anti-D (Figure 4A and 4C).

These results were confirmed using flourescein isothiocyanate (FITC)-labelled sensitized RBC and purified blood monocytes. In this experiment, erythrocytes were labelled with FITC, sensitized with anti-D, with and without treatment with EndoS. After washing the cells with PBS and removal of uningested RBC by hypotonic buffer, the degree of ingested RBC was determined by measuring the fluorescence. This experiment showed a significant decrease in phagocytosis of RBC sensitized with EndoS pre-treated anti-D (Figure 4B). This clearly shows that the pre-treatment of anti-D with EndoS before sensitisation of RBC abrogates the phagocytosis enhancing activity of anti-D and decreases the accumulation of intracellular hemoglobin in monocytes. Thus, whilst not preventing therapeutic efficacy, modifying glycosylation does reduce or eliminate unwanted side-effects.

### EndoS inhibits hemolysis of sensitized red blood cells

Anti-D treatment does not activate complement pathway and RBC coated with this antibody are rather cleared by mononuclear phagocytic cells via Fcgamma receptors. Therefore, in attempt to study hemolytic effects or complement activation of sensitized RBC, polyreactive rabbit anti-human RBC IgG were used. Also in this case the amount of IgG on sensitized RBC and proper hydrolysis of IgG glycan by EndoS was confirmed by Western blot and ELISA (Figure 5A, 5B, 5C). Thus, the rabbit anti-human RBC, with and without treatment with modification of glycoslation, was added to whole blood.

After pelleting the blood cells, the supernatants were analyzed for haemoglobin using DAF. The results present a clear increase in haemoglobin concentration using sensitized RBC. In contrast, only negligible DAF activity was seen using EndoS-treated anti-RBC, indicating inhibition of hemolysis (Figure 6A).

It was next examined whether a simultaneous addition of EndoS and antibody to blood, without previous treatment of antibody with EndoS, could have the similar anti-hemolytic effect. It was clearly shown that pre-treatment of antibody by EndoS is not a prerequisite for inhibition of hemolysis and that a direct addition of EndoS to blood was sufficient to exclude hemolytic effect of anti-RBC (Figure 6A). EndoS by itself did not have any effect on haemolysis (Figure 6A). Furthermore, addition of increasing concentration of rabbit anti-human RBC IgG to whole blood caused a severe agglutination of RBC and leukocytes. Remarkably, addition of EndoS-treated antibody abrogated this effect (Figure 6B). In conclusion, modification of glycosylation achieves anti-haemolytic activities as demonstrated by pre-treatment of anti-RBC with enzyme.

### EndoS effectively prevents binding of C1q to IgG-sensitized RBC

Human RBC were sensitized with rabbit anti-human RBC IgG, with or without pre-treatment of antibody with EndoS. Pellets of sensitized RBC were then incubated with human serum for 15 minutes at 30°C followed by detection of complement factor C1q uptake using FITC labelled anti-human C1q.

There was a significant decreased fluorescence intensity in the sample containing RBC sensitized with EndoS-treated anti-RBC, compared to RBC treated with native antibody, that indicated the inability of C1q to bind to these RBC (Figure 7A). These results provide evidence for complement inhibitory/anti-hemolytic properties of EndoS.

### EndoS inhibits IL-8 secretion by monocytes induced by sensitized RBC

*In vitro* cytokine production reveals the *in vivo* activity of sensitized RBC/autoantibodies predicting the extent of hemolysis and harmfulness of autoantibodies. It has been reported that interleukin-8 (IL-8) besides being produced by monocytes in response to lipopolysaccharide, tumor necrosis factor-alpha or IL-1 also has been implicated as an indicator of red blood cell incompability and mediator of the pathological events in hemolysis as for example in haemolytic transfusion reactions (Davenport et al (1994) Transfusion, 34:297-303, Davenport et al (1990) Blood, 76:2439-2442 and Wikman et al (2005) Ann Hematol., 84:150-158).

Experiments were performed to establish the role of EndoS regarding the *in vitro* response of monocytes exposed to sensitized RBC by measuring the release of IL-8. Thus, monocytes were incubated with anti-D sensitized RBC and the secretion of IL-8 was measured using ELISA. A significantly elevated IL-8 concentration was observed in both THP-1 cells and peripheral blood monocytes. Noticeably, RBC sensitized with EndoS-treated anti-D showed significantly decreased secretion of IL-8 in THP-1 cells and completely abolished IL-8 secretion in blood monocytes (Figure 7B).

### EndoS inhibits oxygen metabolite production by monocytes due to exposure to sensitized RBC

To further explore the effects of EndoS on monocyte-driven cell cytotoxicity to sensitized RBC the release of reactive oxygen species using a chemiluminescent test (CLT) was measured. This method allows differentiation of the clinically significant antibodies from clinically benign and is used to predict the clinical outcome of autoantibody in transfusion of incompatible blood or severity of hemolytic disease of the newborn (Hadley et al (1999) Pediatr Nephrol., 17:91-96).

RBC were opsonised with immune plasma from three patients with positive direct antiglobulin tests (DATs) antigen. Monocytes responses to sensitized red cells were compared with their response to unsensitized cells and expressed as a ratio (opsonic index). RBC incubated with immune plasma from these patients gave positive results in CLT with opsonic index =/>2.5. In contrast, monocyte responses to RBC incubated with immune plasma pre-treated with EndoS were efficiently decreased and in some cases even below the control plasma (Figure 8). These results indicate that treatment of immune plasma with EndoS completely abrogates *in vitro* cytotoxic activity of monocytes.

### Discussion

The results obtained shows how undesirable side-effects of therapeutic antibodies can be abrogated and particular negative effects of autoantibodies decreased. They demonstrated that modification of glycosylation changes the way the antibody intereacts with the immune system. Using anti-D antibodies as a model the destructive processes of opsonized RBC was eliminated by modification of glycosylation via EndoS. The results clearly demonstrate that anti-D with modified glycosylation counteracts Fc gamma receptor mediated phagocytosis of anti-D coated RBC, inhibits binding of C1q component of complement to IgG-coated RBC and minimizes cytotoxic effects of IgG anti-RBC autoantibodies, as well as oxygen radicals and secretion of IL-8 cytokine. This indicates that modification of glycosylation possesses the ability to protect RBC from lysis, predominantly extravascularly, but also intravascularly in the blood vessels, whilst not preventing the antibody acting therapeutically.

The action of anti-D or intravenous immune globulin (IVIG) in ITP is thought to involve extravascular hemolysis of anti-D sensitized RBC by macrophages in the spleen which causes decreased sequestration of autoantibody sensitized platelets. However, anti-D or IVIG administrated for treatment of immune thrombocytopenic purpura (ITP) may cause severe complications such as acute hemolysis, disseminated intravascular coagulation (Gaines et al (2005) Blood, 106:1532-1537 and Gaines et al (2000) Blood 95:2523-2529) and acute renal failure (Kees-Folts et al (2002) Pediatr Nephrol., 17:91-96).

Here we show that the treatment of anti-D with EndoS to modify glycosylation, before injection, extends the survival of Ig-G-coated RBC in the circulation providing a simple, effective approach. Thus, negative side-effects of therapeutic antibodies can be reduced through modification of glycosylation.

One benefit of EndoS action is the specific activity against antibodies, without affecting RBC in binding to the cell surface or interfering with group antigens or membrane proteins (data not shown). Other endoglycosidases as EndoF from *Elisabethkingiameningoseptica* or EndoE from *Enteroccocus faecalis* have activity on a broad range of glycoproteins and may be employed.

The results presented here show the invention provides an effective approach to improve therapeutic antibodies and the way such antibodies interact with the immune system. The specific case of anti-D provides an effective model, it shows abroad biological significance not only regarding autoimmune haemolytic anemia, but also other pathological conditions characterized by an abnormal removal of antibody opsonized RBC by macrophages.

### Example 5 - Monoclonal antibodies with modified glycosylation dominantly suppress the induction of arthritis by a cocktail of two arthritogenic mAbs (M2139/CIIC1)

Groups of 4 months old male QB = (BALB/c x B10.Q)F1 mice were injected intravenously with a mixture either consisting of:
i) M2139D (2mg) + CIIC1D (2 mg); or
ii) UL1D (2 mg) + CIIC2D (2 mg)
After 15 minutes the mice were injected (i.v.) with 9 mg of the cocktail of two arthritogenic anti-CII mAbs (M2139, 4.5 mg + CIIC1, 4.5 mg). On day 5, all the mice received 25 µg/i.p./mouse of lipopolysaccharide (LPS) from E.coli 055:B5. Mice were monitored for arthritis development on indicated days.

Thus, mice receiving treatment (i) were treated with EndoS-treated antibodies recognising epitopes which overlap with the epitopes recognised by the arthritogenic antibodies. Mice receiving treatment (ii) were treated with EndoS-treated antibodies recognising epitopes which do not overlap with the epitopes recognised by the arthritogenic antibodies.

Figure 9 shows arthritis incidence in panel A. Mean arthritis score, i.e. disease severity is shown in panel B for arthritic animals only. n indicates the number of mice used in each group. Error bars indicate ± SEM. As can be seen from the results depicted, using EndoS treated antibodies to overlapping or non-overlapping epitopes, suppresses arthritis in this model of rheumatoid arthritis. Thus, EndoS treated antibodies can dominantly suppress CAIA, i.e. suppression is separate from direct competition for epitopes.

### Example 6 - Arthritis suppression by antibodies with modified glycosylation is independent of the spleen

Groups of 3 months old male QB = (BALB/c x B10.Q)F1 mice were either spleenectomized or sham operated. After one month, mice were either injected intravenously with a mixture consisting of the EndoS treated antibodies M2139D (2mg) + CIIC1D (2 mg), or left untreated. Subsequently they were injected with 9 mg of the cocktail of two arthritogenic anti-CII mAbs (M2139, 4.5 mg + CIIC1, 4.5 mg) = day 0. 25 µg/i.p./mouse of lipopolysaccharide (LPS) from E.coli 055:B5 was injected to all of the mice to enhance arthritis incidence and severity.

Figure 10 shows arthritis incidence in panel A. Mean arthritis score, i.e. disease severity is shown in panel B for arthritic animals only. n indicates the number of mice used in each group. Error bars indicate ± SEM. As can be seen from the results depicted, animals which were not treated with EndoS treated antibodies experienced disease at equivalent levels of incidence and severity regardless of the presence or absence of a spleen. By contrast, using EndoS treated antibodies suppresses arthritis in this model regardless of the presence or absence of spleen. Thus, the suppression of arthritis by these antibodies is spleen-independent.

### Example 7 - Arthritis suppression by antibodies with modified glycosylation is independent of the antigen specificity

Groups of 4 months old male QB = (BALB/c x B10.Q)F1 mice were either injected intravenously with a mixture consisting of the EndoS treated antibodies Hy2.15D (2mg) + L234D (2 mg), or left untreated. Subsequently they were injected with 9 mg of the cocktail of two arthritogenic anti-CII mAbs (M2139, 4.5 mg + CIIC1, 4.5 mg) = day 0. 25 µg/i.p./mouse of lipopolysaccharide (LPS) from E.coli 055:B5 was injected to all of the mice to enhance arthritis incidence and severity. Hy2.15D is a monoclonal mouse IgG1, reactive with TNP. L234D is a monoclonal mouse IgG2a, reactive with HLA-DR.

Figure 11 shows arthritis incidence in panel A. Mean arthritis score, i.e. disease severity is shown in panel B for arthritic animals only. n indicates the number of mice used in each group. Error bars indicate ± SEM. As can be seen from the results depicted, arthritis incidence and severity is suppressed using EndoS treated antibodies which are specific for completely different antigen targets to the disease-inducing antibodies. Thus, EndoS treated antibodies can dominantly suppress CAIA, i.e. suppression is separate independent of antigen specificity.

### Example 8 - A single antibody with modified glycosylation suppresses arthritis at low dose

Groups of 4 months old male QB = (BALB/c x B10.Q)F1 mice were either injected intravenously with different concentrations (50, 250, 1000 or 4000 µg) of a single EndoS treated Ab (M2139D), or left untreated. Subsequently they were injected with 9 mg of the cocktail of two arthritogenic anti-CII mAbs (M2139, 4.5 mg + CIIC1, 4.5 mg) = day 0. 25 µg/i.p./mouse of lipopolysaccharide (LPS) from E.coli 055:B5| was injected to all of the mice to enhance arthritis incidence and severity.

Figure 12 shows arthritis incidence in panel A. Mean arthritis score, i.e. disease severity is shown in panel B for arthritic animals only. n indicates the number of mice used in each group. Error bars indicate ± SEM. As can be seen from the results depicted, arthritis incidence and severity is suppressed using a single EndoS treated antibody at a dose as low as 250µg.

## Claims

1. An intravenous immunoglobulin (IVIG) preparation which has been treated with an EndoS enzyme wherein the glycosylation of the EndoS-treated IVIG preparation consists only of the first N-acetylglucosamine residue with an optional fucose group.

2. An *in vitro* method of producing an intravenous immunoglobulin (IVIG) preparation according to claim 1 comprising treating an IVIG preparation with an EndoS enzyme.

## Patentansprüche

1. Intravenöses Immunglobulin(IVIG)-Präparat, das mit einem EndoS-Enzym behandelt worden ist, wobei die Glycosylierung des EndoS-behandelten IVIG-Präparats nur aus dem ersten N-Acetylglucosaminrest mit einer optionalen Fucosegruppe besteht.

2. In-vitro-Verfahren zur Herstellung eines intravenösen Immunglobulin(IVIG)-Präparates gemäß Anspruch 1, das Behandeln eines IVIG-Präparates mit einem EndoS-Enzym umfasst.

## Revendications

1. Préparation d'immunoglobuline pour intraveineuse (IGIV) qui a été traitée avec une enzyme EndoS, de laquelle préparation IGIV traitée avec une enzyme EndoS la glycosylation ne consiste qu'en le premier résidu de N-acétyl-glucosamine, avec, en option, un groupe fucose.

2. Procédé de production *in vitro* d'une préparation d'immunoglobuline pour intraveineuse (IGIV) conforme à la revendication 1, comprenant le fait de traiter une préparation IGIV avec une enzyme EndoS.
